# EUROPEAN PATENT SPECIFICATION

(11) **EP 2 610 352 B1**
(45) Date of publication and mention of the grant of the patent: **08.10.2014**
(21) Application number: 13155136.8
(22) Date of filing: 16.02.2010
(51) Int. Cl.: C12Q 1/68

(54) **Template-independent ligation of single-stranded DNA**
Vorlagenunabhängige Ligation von Einzelstrang-DNA
Ligature indépendante de la matrice d'un ADN simple brin

(30) Priority: 16.02.2009 US 152868 P
(43) Date of publication of application: 03.07.2013
(62) Divisional of application: 10741881.6
(73) Proprietor: Epicentre Technologies Corporation, Madison, WI 53719 (US)
(72) Inventor: Jendrisak, Jerome, Middleton, WI 53562-2345 (US); Decker, Joanne, Cambridge, WI 53523 (US); Dahl, Gary, Madison, WI 53726 (US)
(74) Representative: Murphy, Colm Damien

(56) References cited:
- WO-A2-2007/106509
- BLONDAL T ET AL: "Isolation and characterization of a thermostable RNA ligase 1 from a Thermus scotoductus bacteriophage TS2126 with good single stranded DNA ligation properties", NUCLEIC ACIDS RESEARCH, OXFORD UNIVERSITY PRESS, SURREY, GB, vol. 33, no. 1, 1 January 2005 (2005-01-01), pages 135-142, XP003026551, ISSN: 0305-1048, DOI: 10.1093/NAR/GKI149
- WEISS B ET AL: "Ezymatic breakage and joining of deoxyribonucleic acid. VII. Properties of the enzyme-adenylate intermediate in the polynucleotide ligase reaction", JOURNAL OF BIOLOGICAL CHEMISTRY, AMERICAN SOCIETY FOR BIOCHEMISTRY AND MOLECULAR BIOLOGY, US, vol. 243, no. 17, 10 September 1968 (1968-09-10), pages 4556-4563, XP002677345, ISSN: 0021-9258
- NUNEZ ET AL: "Application of Circular Ligase to Provide Template for Rolling Circle Amplification of Low Amounts of Fragmented DNA", 19TH INTERNATIONAL SYMPOSIUM ON HUMAN IDENTIFICATION - 2008, , 2008, pages 1-7, XP008153466, Retrieved from the Internet: URL:http://www.promega.com/~/media/files/r esources/conference%20proceedings/ishi%201 9/oral%20presentations/nunez.pdf?la=en [retrieved on 2010-03-31]
- C. Torchia ET AL: "Archaeal RNA ligase is a homodimeric protein that catalyzes intramolecular ligation of single-stranded RNA and DNA", Nucleic Acids Research, vol. 36, no. 19, 27 September 2008 (2008-09-27), pages 6218-6227, XP055110097, ISSN: 0305-1048, DOI: 10.1093/nar/gkn602

## Description

This application claims priority to U.S. Provisional Application Ser. No. 61/152,868, filed Feb. 16, 2009.

### FIELD OF THE INVENTION

The invention relates to improved ligation reaction mixtures and methods for template-independent intramolecular ligation (e.g., circularization) of single-stranded DNA (ssDNA) or single-stranded RNA (ssRNA) molecules using a thermostable RNA ligase, particularly for circularization of ssDNA molecules in a population of ssDNA molecules of unknown sequence and/or size, and kits therefore, and methods of use and applications thereof.

### BACKGROUND OF THE INVENTION

It has long been known in the art that bacteriophage T4 RNA ligase I (Rnl1), which infects *Escherichia coli,* has template-independent intramolecular ligation activity (Gumport and Uhlenbeck, in Gene Amplification and Analysis, Vol. II: Analysis of Nucleic Acid Structure by Enzymatic Methods, Chirikjian and Papas, eds. Elsevier North Holland, Inc., 1980; McCoy and Gumport, Biochemistry 19: 635-642, 1980; Sugino, A et al., J Biol Chem 252: 1732-1738, 1977), but this activity is far too low and inefficient for practical use in generating circular ssDNA molecules from linear ssDNA molecules. Without being bound by theory, this may be explained, at least in part, by the fact that, although T4 RNA ligase can use a 5'-phosphorylated end of a ssDNA molecule as a "ligation donor" (or "donor") approximately as well as the 5'-phosphorylated end of a ssRNA molecule, the 3'-hydroxyl end of a ssDNA molecule is used as a "ligation acceptor" (or "acceptor") much less efficiently than the 3'-hydroxyl end of RNA.

U.S. Patent No. 7,303,901 and Blondal et al. (Nucleic Acids Res 33: 135-142, 2005), disclosed a thermostable RNA ligase derived from the Thermus bacteriophage TS2126, which infects the thermophilic bacterium *Thermus scotoductus.* This enzyme, referred to herein as "bacteriophage TS2126 thermostable RNA ligase" or "phage TS2126 RNA ligase," or more simply, as "TS2126 RNA ligase," has good template-independent intramolecular ligation activity for linear ssDNA as well as ssRNA substrates that have 5' phosphoryl and 3' hydroxyl groups. Bacteriophage TS2126 RNA ligase is significantly more thermostable than those of mesophilic RNA ligases such as the T4 RNA ligase, being stable at up to about 70 degrees C. The range of temperature for TS2126 RNA ligase activity can be greater than about 40 degrees C, for example, from about 50 degrees C to about 75 degrees C. The enhanced thermostability of bacteriophage TS2126 RNA ligase allows it to be used under temperature conditions which would be prohibitive for other enzymes. For example, due to its thermostability, TS2126 RNA ligase enzyme can be used at higher temperatures which reduce undesirable secondary structures of ssDNA or ssRNA. This enzyme was commercially available for about six years (as of the filing date of the present patent application) from the company Prokaria (Reyjkavik, Iceland) under the trademarks of THERMOPHAGE™ RNA ligase II or THERMOPHAGE™ ssDNA ligase, and since mid-December, 2008, was available under the trademark of THERMOPHAGE™ ssDNA ligase, a Prokaria brand from Matis (www.matis.is; Reyjkavik, Iceland) and from Prokazyme (www.prokazyme.com; Reyjkavik, Iceland). In addition, EPICENTRE Biotechnologies, a company in Madison, Wisconsin, USA that employs the present applicants, has sold bacteriophage TS2126 RNA ligase since December, 2004 under the trademark of CIRCLIGASE™ ssDNA ligase, and has devoted considerable efforts to studying its properties, reaction conditions, and uses.

Torchia, C et al. (Nucleic Acids Res 36: 6218-6227, 2008), disclosed that an RNA ligase derived from thermophilic archeabacteria (e.g., Methanobacterium thermoautotrophicum RNA ligase 1 or "MthRnl") has template-independent ligase activity in circularizing linear ssDNA molecules.

Methods that use a template-independent ligase for intramolecular template-independent ligation (i.e., cirularization) of ssDNA molecules are known in the art. For example, U.S. Patent Application No. 20060240451 disclosed methods for ligating linear first-strand cDNA molecules prepared from 5'-end fragments of mRNA using bacteriophage TS2126 RNA ligase, and then amplifying the circular first-strand cDNA molecules by rolling circle replication (RCR) or rolling circle transcription (RCT).

U.S. Patent Application No. 20040197802 disclosed a sense promoter primer comprising a 5'-end portion and a 3'-end portion, wherein the 5'-end portion exhibits a sense RNA polymerase promoter sequence and the 3'-end portion exhibits a sequence that is complementary to a sequence in a target nucleic acid in a biological sample. They also disclosed a method comprising circularizing the linear sense promoter-containing first-strand cDNA by template-independent intramolecular ligation using phage TS2126 RNA ligase. In some embodiments, the method further comprised generating a circular transcription substrate, and transcribing the circular transcription substrate. This method is useful for amplifying the mRNA molecules in a biological sample.

A. Polidoros et al. (BioTechniques 41: 35-39, 2006) described use of the template-independent TS2126 RNA ligase (CIRCLIGASE™ ssDNA ligase, (EPICENTRE Biotechnologies, Madison, WI, USA) as a step in a method for amplifying cDNA ends for random amplification of cDNA ends (RACE).

U.S. Patent Application No. 20080242560 of Gunderson KL and Steemers, F disclosed use of CIRCLIGASE™ ssDNA ligase (EPICENTRE) in methods comprising: making digital DNA balls (e.g., FIG 8 in U.S. Patent Application No. 20080242560); and/or locus-specific cleavage and amplification of DNA, such as genomic DNA, including for amplification by multiple displacement amplification or whole genome amplification (e.g., FIG 17 therein) or by hyperbranched RCA (e.g., FIG 18 therein) for generating amplified nucleic acid arrays (e.g., ILLUMINA BeadArrays™; ILLUMINA, San Diego CA, USA).

U.S. Patent Application Nos. 20090011943; 20090005252; 20080318796; 20080234136; 20080213771; 20070099208; and 20070072208 of Drmanac et al. disclosed the use of CIRCLIGASE™ ssDNA ligase (EPICENTRE) to generate circular ssDNA templates for massively parallel DNA sequencing; for example, U.S. Patent Application Nos. 20090011943 disclosed use of a ligation reaction mixture for template-independent ligation (i.e., circularization) that contained final concentrations of: 10 units per microliter of CIRCLIGASE™ ssDNA ligase, 50 mM MOPS, pH, 7.5, 10 mM KCl, 5 mM MgCl₂, 1 mM DTT, 25 micromolar ATP, 1.25 mM MnCl₂, 10% PEG4000 and 0.5-10 picomoles per microliter of ssDNA.

Nunez, AN et al. (Nunez, AN, Kavlick, MF, Robertson, JM, and Budowle, B, "Application of Circular Ligase to Provide Template for Rolling Circle Amplification of Low Amounts of Fragmented DNA", 19th International Symposium on Human Identification, October 13-16, 2008, Hollywood, California) described yet another use for ssDNA molecules generated using CIRCLIGASE™ ssDNA ligase (EPICENTRE) in:
http://www.promega.com/geneticidproc/ussymp19proc/oralpresentations/Nunez.pdf. These researchers at the Federal Bureau of Investigation disclosed methods comprising: denaturing degraded DNA samples to obtain ssDNA fragments; ligating the linear ssDNA fragments with CIRCLIGASE ssDNA ligase to obtain circular ssDNA fragments; and then amplifying the circular ssDNA fragments using degenerate primers and phi29 DNA polymerase by RCA. This method has the potential for whole genome amplification (WGA) of degraded DNA samples by a RCA mechanism, which could provide significant benefits for forensic analysis of compromised biological evidence or other applications. The investigators developed what they believed to be optimal conditions for circularizing linear ssDNA molecules and laid the foundations for typing degraded DNA samples which could not previously be typed. However, even with the reaction conditions that they considered optimal, they noted that the amount of ligated product was variable and seemed to depend on the sequence, since they observed that an oligonucleotide with a 5' G and a 3' T nucleotide ligated significantly better than its complementary oligonucleotide with a 5' A and a 3' C under identical ligation conditions.

Thus, although bacteriophage TS2126 thermostable RNA ligase (sold under the trademarks of THERMOPHAGE™ RNA ligase II or THERMOPHAGE™ ssDNA ligase by the companies Prokaria, Matis, and Prokazyme, Reyjkavik, Iceland) and under the trademark CIRCLIGASE™ ssDNA ligase by EPICENTRE Biotechnologies, Madison, Wisconsin, USA) is the most efficient ligase known for template-independent intramolecular ligation of ssDNA molecules (based on the citations discussed herein and the personal observations of the applicants and their colleagues), a persistant and, until now, intractable problem during several years has been the variable intramolecular ligation efficiency of the enzyme for linear ssDNA molecules with different sequences and sizes. Thus, the present applicants and their colleagues at EPICENTRE, as well as other researchers like Nunez et al., discussed above, have observed quite different levels of intramolecular ligation of oligodeoxyribonucleotides with linear ssDNA substrates of identical or very similar sizes but with even small differences in nucleotide sequences, or with different sizes, ranging from polynucleotides of less than 100 bases to kilobases in size. For example, in some cases, even a single nucleotide difference in an oligodeoxyribonucleotide sequence resulted in a large difference in intramolecular ligation efficiency. If much higher proportions of different linear ssDNA molecules could be intramolecularly ligated and they could be ligated to the same relative extent, much better experimental results would be obtained and much better conclusions could be made. Thus, what is greatly needed in the art since the discovery of the bacteriophage TS2126 thermostable RNA ligase are new improved ligation methods, ligation reaction mixtures, and kits that permit much higher levels of linear ssDNA molecules to be intramolecularly ligated, and that permit all of them to be intramolecularly ligated to the same relative extent, regardless of their respective nucleotide sequences or sizes. If such improved ligation methods, ligation reaction mixtures, and kits could be found, what is further needed are new methods that use the improved ligation methods, ligation reaction mixtures, and kits to make improved methods for amplifying nucleic acid molecules for gene expression analysis by quantitative endpoint PCR or real-time PCR, or methods for relative gene expression analysis by hybridization to arrays or microarrays or by sequencing using massively parallel DNA sequencing (i.e., so-called "next-generation" DNA sequencing), or methods for RACE as described by Polidoros et al. (discussed above), or methods for genomic DNA amplification by RCA-WGA as described by Nunez et al. (discussed above), or methods for genomic DNA amplification (including locus-specific amplification) and/or sequencing as described by Gunderson KL and Steemers, F and by Drmanac et al. (both discussed above), or methods for any other application for which intramolecular ligation of ssDNA or the resulting circular ssDNA molecules are employed. For example, some other applications and methods in which CIRCLIGASE™ ssDNA ligase has been used are presented by Shroff, H et al. (Nano Letters 5: 1509, 2005; and Biophysical Journal 94: 2179, 2008); Lin, C et al. (Angewandte Chemie 118: 7699, 2006); Korlach, J et al. (Proc. Natl. Acad. Sci. USA 105: 1176, 2008); McArthur, M and Bibb, MJ (Proc. Natl. Acad. Sci. USA 105: 1020, 2008); and Kuhn, H and Frank-Kamenetskii, MD (Nucleic Acids Res 36: e40, 2008). New improved ligation reaction mixtures, improved ligation reaction conditions, and methods that permit more efficient intramolecular ligation and that permit all of ssDNA molecules to be intramolecularly ligated to the same relative extent, regardless of their respective nucleotide sequences or sizes, are needed and would beneficial for all of these methods.

In short, what is needed are improved ligation reaction mixtures and methods that permit high and consistent levels of intramolecular ligation of ssDNA molecules of different sequences and sizes using thermostable RNA ligases, such as bacteriophage TS2126 RNA ligase. The present application discloses new improved ligation reaction mixtures and methods that solve this long-standing problem, and new processes that employ those new improved template-independent intramolecular ligation reaction mixtures and methods.

### SUMMARY OF THE INVENTION

One embodiment of the present invention is a ligation reaction mixture for template-independent intramolecular ligation of linear ssDNA molecules comprising:
(a) linear ssDNA molecules that have 5'-phosphoryl and 3'-hydroxyl groups;
(b) a composition of thermostable RNA ligase molecules, wherein a high proportion of the thermostable RNA ligase molecules are adenylated and wherein the concentration of the adenylated thermostable RNA ligase molecules in the ligation reaction mixture equals or exceeds the molarity of the ssDNA molecules;
(c) a buffer that maintains the final pH at between about pH 6.5 and about 8.0; and
(d) a manganese salt at a concentration that is optimal for the thermostable RNA ligase, wherein the final concentration of Mn²⁺ cations in the ligation reaction mixture is between 0.5 and 10 mM;
wherein, ATP is either not present in the reaction buffer or is present at a molar concentration that is less than the concentration of the non-adenylated form of the thermostable RNA ligase.

In some preferred embodiments, no ATP is added to the ligation reaction mixture.

The ligation reaction mixture of the invention additionally comprises betaine (zwitterionic trimethylglycine) at a final concentration of 0.25 to 2 molar. In some preferred embodiments the concentration of betaine in the ligation reaction mixture is about 1 M.

In some preferred embodiments, Mg2+ cations are not added to the ligation reaction mixture.

The invention also comprises methods for using the improved ligation reaction mixture for template-independent intramolecular ligation of linear ssDNA molecules to synthesize circular ssDNA molecules using a thermostable RNA ligase. For example, one method comprises:
1. Preparing a ligation reaction mixture comprising: (a) linear ssDNA molecules that have 5'-phosphoryl and 3'-hydroxyl groups; (b) a composition of thermostable RNA ligase molecules, wherein a high proportion of the thermostable RNA ligase molecules are adenylated and wherein the concentration of the adenylated thermostable RNA ligase molecules in the ligation reaction mixture equals or exceeds the molarity of the ssDNA molecules; (c) a buffer that maintains the final pH at between about pH 6.5 and about 8.0; and (d) a manganese salt at a concentration that is optimal for the thermostable RNA ligase, wherein the final concentration of Mn²⁺ cations in the ligation reaction mixture is between 0.5 and 10 mM; wherein, ATP is either not present in the reaction buffer or is present at a molar concentration that is less than the concentration of the non-adenylated form of the thermostable RNA ligase; and
2. Incubating the linear ssDNA molecules in the ligation reaction mixture at a reaction temperature between about 40 degrees C and about 70 degrees C for sufficient time wherein circular ssDNA molecules are synthesized.

In some preferred embodiments of the method, no ATP is added to the ligation reaction mixture.

In all embodiments of the method of the invention, the ligation reaction mixture additionally comprises betaine (zwitterionic trimethylglycine) at a final concentration of 0.25 to 2 molar. In some preferred embodiments the concentration of betaine in the ligation reaction mixture is about 1 M.

In some preferred embodiments of the method, Mg2+ cations are not added to the ligation reaction mixture.

In some preferred embodiments of the method, the template-independent thermostable RNA ligase is selected from the group consisting of: a *Thermus* bacteriophage RNA ligase: bacteriophage TS2126 RNA ligase; an archaebacterium RNA ligase; *Methanobacterium thermoautotrophicum* RNA ligase 1.

In one preferred embodiment, the method comprises:
1. Preparing a ligation reaction mixture comprising:
   (a) the linear ssDNA molecules that have 5'-phosphoryl and 3'-hydroxyl groups;
   (b) a composition of phage TS2126 thermostable RNA ligase, wherein ≥60% of the thermostable RNA ligase molecules are adenylated and are present in an amount wherein the molarity of the adenylated RNA ligase molecules in the ligation reaction mixture equals or exceeds the molarity of the linear ssDNA molecules (e.g., a concentration of about 1 micromolar of the adenylated RNA ligase molecules for a concentration of about 0.5 micromolar of the linear ssDNA molecules); and
   (c) the buffering agent (e.g., TRIS acetate at a final pH in the ligation reaction mixture of about pH 7.8);
   (d) the manganese salt that provides the Mn²⁺ cations at a final concentration of between about 0.5 and 10 mM (e.g., at a final concentration of about 2.5 mM of Mn²⁺ cations); and
2. Incubating the ligation reaction mixture at a temperature between about 40 degrees C and about 70 degrees C (e.g. about 55 degrees C and about 65 degrees C; e.g., about 60 degrees C) for sufficient time wherein circular ssDNA molecules are generated from the linear ssDNA molecules. In all embodiments of this method, the ligation reaction mixture additionally contains betaine (zwitterionic trimethylglycine) at a final concentration of about 0.25 to 2 molar.

In some embodiments of any of the above methods, the linear ssDNA molecules that have 5'-phosphoryl and 3'-hydroxyl groups for intramolecular ligation comprise or consist of a population of linear ssDNA molecules wherein the nucleotide sequences of the 5' or 3' ends are unknown and/or wherein the linear ssDNA molecules vary in size.

In some preferred embodiments of the method, the linear ssDNA molecules that have 5'-phosphoryl and 3'-hydroxyl groups used in the method for intramolecular ligation comprise or consist of a population of linear first-strand cDNA molecules generated by extension of one or more first-strand cDNA synthesis primers that anneal to complementary sequences exhibited by one or more target nucleic acid molecules in a biological sample using a DNA polymerase.

In some preferred embodiments, the linear first-strand cDNA molecules generated by extension of one or more first-strand cDNA synthesis primers, are further purified by removing the target nucleic acid molecules using a nuclease that specifically digests the target nucleic acid molecules but not the linear first-strand cDNA molecules, or by selectively purifying the linear first-strand cDNA molecules from the target nucleic acid molecules by incorporating an affinity tag into the linear first-strand cDNA molecules and pulling them out using an affinity binding substance with which the affinity tag forms a specific binding pair, which affinity binding substance is attached to a surface.

For example, in some embodiments of the method wherein the linear first-strand cDNA molecules used in the method are generated from RNA molecules in a biological sample, the nuclease that specifically digests the target nucleic acid molecules but not the linear first-strand cDNA molecules is selected from among RNase H, and a combination of both RNase I and RNase III. In some embodiments wherein the linear first-strand cDNA molecules used in the method are generated from DNA molecules in the biological sample, the nuclease that specifically digests the target nucleic acid molecules but not the linear first-strand cDNA molecules is, for example, a single-strand-specific DNase that specificially digests only the DNA target nucleic acid molecules.

In some embodiments of the method comprising incorporating an affinity tag into the linear first-strand cDNA molecules and pulling out the primer extension products using an affinity binding substance, the affinity tag comprises a biotin moiety (e.g., joined to one or more nucleotides in the first-strand cDNA molecules) and the affinity binding substance comprises streptavidin which is attached to a surface.

In some preferred embodiments of the method wherein the linear ssDNA molecules used for intramolecular ligation are prepared by extending one or more first-strand cDNA synthesis primers, each of the one or more first-strand cDNA synthesis primers comprises: a 5'-end portion comprising or consisting of a tag that exhibits a sequence that is not substantially complementary to a sequence in the target nucleic acid molecules; and a 3'-end portion that exhibits a sequence that is complementary to a sequence exhibited by the at least one target nucleic acid molecules from a biological sample; and tagged circular ssDNA molecules are synthesized. By the statement "a 3'-end portion that exhibits a sequence that is complementary to a sequence exhibited by the at least one target nucleic acid molecules from a biological sample," we mean herein that the 3'-end is complementary to either sequences exhibited by the target nucleic acid molecules themselves or to sequences that are joined to a 3'-end of the target nucleic acid molecules from the biological sample (such as a poly(A) or other homopolymeric tail that is added to the target nucleic acid molecules using an *in vitro* nucleic acid modification reaction; e.g., using poly(A) polymerase). For example, in some embodiments, the linear ssDNA molecules for intramolecular ligation for use in the method are prepared from nucleic acid molecules of interest in a biological sample, wherein said nucleic acid molecules of interest have been modified (e.g., by poly(A) tailing of RNA with poly(A) polymerase, or tailing of DNA with terminal deoxynucleotidyl transferase, or ligation of an adapter oligonucleotide to the 3'-ends of DNA or RNA molecules of interest, or addition of a 3'-terminal sequence to DNA or RNA molecules of interest using the terminal tagging method disclosed in U.S. Patent Application Nos. 20050153333 or 20090227009. Thus, the method also includes use of linear ssDNA molecules in the method which have been prepared from nucleic acids from a biological sample which have been modified.

In some preferred embodiments wherein the one or more first-strand cDNA synthesis primers comprises or consists of a tag in the 5'-end portion, the tag comprises or consists of one or more tag domains selected from the group consisting of: an RNA polymerase promoter tag domain that exhibits a sense promoter sequence; cleavage site tag domains; sequencer-specific sequencing tag domains; capture tag domains; amplification tag domains; detection tag domains; and address tag domains.

The tag in the 5'-portions of the first-strand cDNA synthesis primer can comprise any desired tag domains and can exhibit any desired sequences for any desired purpose. For example, in some embodiments the 5' portion comprises a tag that comprises one to more sequencing tag domains that exhibit the sequences of the Roche 4FLX 54A and 454B sequencing tags and, after isolating the fragments in the desired size range, they are used as templates for next-generation using the Roche 454 Genome Sequencer FLX System. Similarly, in other embodiments, the 5'- and 3'-tagged DNA fragments, after isolating those that are in the desired size range, are used as templates for next-generation using another sequencing platform (e.g., using the ROCHE 454 sequencing platform, the ILLUMINA™ SOLEXA™ sequencing platform, the LIFE TECHNOLOGIES / APPLIED BIOSYSTEMS' SOLID™ sequencing platform, the PACIFIC BIOSCIENCES' SMRT™ sequencing platform, the POLLONATOR Polony sequencing platform, the COMPLETE GENOMICS sequencing platform, the INTELLIGENT BIOSYSTEMS' sequencing platform, or the HELICOS sequencing platform). In some preferred embodiments, the 5'- and 3'-tagged DNA fragments are generated using this method from target DNA comprising a whole genome of a cell or organism.

In some embodiments, a sequencing tag is labeled with an affinity-binding molecule (e.g., biotin) or with a detectable molecule (e.g., a fluorescent dye) that permits capture (e.g., using a surface to which streptavidin is bound for capture of the biotinylated molecules) or detection of 5'- and 3'-tagged DNA fragments that have a tag with the affinity-binding molecule or the detectable molecule at the 5'-end.

In some preferred embodiments of the method wherein the 5'-end portion of the first-strand cDNA synthesis primer comprises or consists of a tag comprising different tag domains, the one or more first-strand cDNA synthesis primers each contains a cleavage site between the tag domains, and the method further comprises the step of: linearizing the circular first-strand cDNA molecules at the cleavage site to obtain linear first-strand cDNA molecules that each exhibit the sequence of the 3'-end portion of the first-strand cDNA synthesis primer at its 5' end and the 5'-end portion of the first-strand cDNA synthesis primer at its 3' end.

For example, in some embodiments, the cleavage site comprises or consists of one or more 2'-deoxyuridine monophosphate (dUMP) moiety or an 8-oxoguanine-2'-deoxyribosyl-monophosphate (8-oxo-dGMP) moiety and the step of linearizing the circular first-strand cDNA molecules at the cleavage site to generate linear first-strand cDNA molecules that each exhibit the sequence of the 3'-end portion of the first-strand cDNA synthesis primer at its 5' end and the 5'-end portion of the first-strand cDNA synthesis primer at its 3' end comprises contacting the circular first-strand cDNA molecules with uracil-N-glycosylase (UNG; EPICENTRE) or 8-oxoguanine DNA glycosylase (Fpg; EPICENTRE), respectively, to generate circular first-strand cDNA molecules that contain one or more abasic sites, and then incubating the circular first-strand cDNA molecules that contain the one or more abasic sites under conditions, such as by incubating in an alkaline solution or in a solution that contains an endonuclease such as endonuclease IV, wherein the circular first-strand cDNA molecules are linearized at or near the abasic sites.

In some preferred embodiments of the method, the first-strand cDNA synthesis primer comprises or consists of a tag in the 5'-end portion that comprises or consists of a two sequencer-specific sequencing tag domains, and the step of linearizing the circular first-strand cDNA molecules at the cleavage site to generate linear first-strand cDNA molecules that each exhibit the sequence of the 3'-end portion of the first-strand cDNA synthesis primer at its 5' end and the 5'-end portion of the first-strand cDNA synthesis primer at its 3' end generates linear ssDNA sequencing templates that have one sequencing tag domain on each of the 5'- and 3'-ends (e.g., di-tagged sequencing templates). In some preferred embodiments, the linear ssDNA sequencing templates that have one sequencing tag domain on each of the 5'- and 3'-ends are used as templates for sequencing on the next-generation sequencer for which the sequencing tag domains are specific.

In some preferred embodiments of the method comprising linearizing the circular first-strand cDNA molecules at the cleavage site, the 5'-end portion of the first-strand cDNA synthesis primer comprises or consists of a tag comprising an RNA polymerase promoter tag domain that exhibits a sense promoter sequence and a cleavage site that is 3'-of the sense promoter sequence, and, following the step of linearizing the circular first-strand cDNA molecules at the cleavage site; the method further comprises the substeps of (i) annealing an oligodeoxyribonucleotide that exhibits an anti-sense promoter sequence to the sense promoter sequence at the 3' end of each of the linear first-strand cDNA molecules generated from said linearizing to generate transcription substrates; and then (ii) transcribing the transcription substrates using an RNA polymerase that binds to the double-stranded RNA polymerase promoter and initiates transcription therefrom. In some preferred embodiments of the method, prior to the substep of transcribing the transcription substrates, the method further comprises the substep of generating double-stranded cDNA by extension of said oligodeoxyribonucleotide that exhibits an anti-sense promoter sequence using a DNA polymerase.

### BRIEF DESCRIPTION OF THE FIGURES

The following figures form part of the present specification and are included to further demonstrate certain aspects of the present invention. The invention may be better understood by reference to one or more of these figures in combination with the detailed description of specific embodiments presented herein.

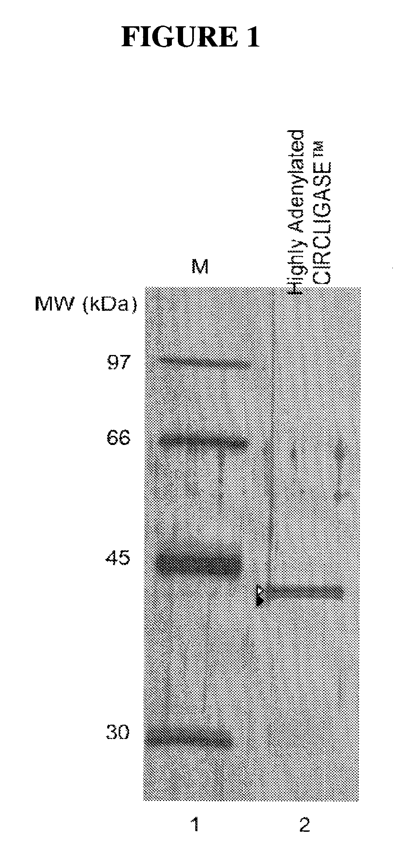

FIG. 1 shows an SDS-PAGE gel of the purified protein obtained from the new clone of phage TS2126 thermostable RNA ligase (also called CIRCLIGASE™ ssDNA Ligase, EPICENTRE) which showed the unexpectedly high level of the adenylated form of the TS2126 RNA ligase (estimated at about 70%) compared to the level of adenylated enzyme in a purified preparation of TS2126 RNA ligase obtained from an older clone (estimated at about 30%). Protein samples were denatured, reduced, and resolved by 10% SDS-PAGE. Protein was visualized by silver staining. Lane 1 shows molecular weight standards (97, 66, 45, 30, 20.1, 14.4 kDa). Lane 2 shows CIRCLIGASE. Open triangles indicate the adenylated enzyme. Filled triangles indicate the non-adenylated enzyme.

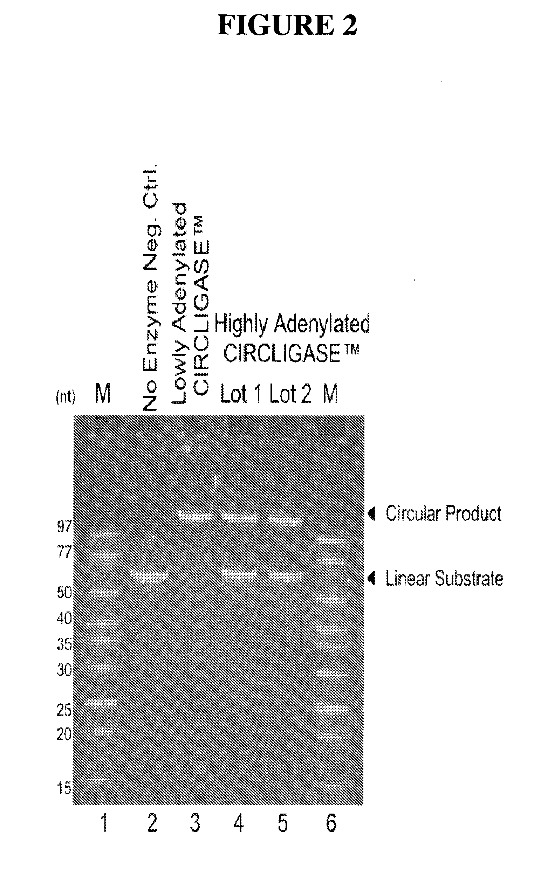

FIG. 2 shows a PAGE analysis of intramolecular ligation activities of the highly adenylated TS2126 RNA ligase (also called CIRCLIGASE™ ssDNA Ligase) obtained using the new clone compared and the lowly adenylated enzyme obtained using the old clone. Unexpectedly, the highly adenylated enzyme was much less active in intramolecular ligation of the linear ssDNA in the Standard Ligation Reaction Mixture than the lowly adenylated enzyme. 1 µg of each CIRCLIGASE enzyme was used for ligation of 10 pmole of the 55-nucleotide Control Oligonucleotide supplied in with CIRCLIGASE™ ssDNA Ligase (EPICENTRE) under standard ligation reaction conditions: (50 mM MOPS pH 7.5, 10 mM KCl,

5 mM MgCl2, 1 mM DTT, 2.5 mM MnCl2, and 50 µM ATP for 1 hour at 60°C). Reactions were stopped by addition of Stop/Load Buffer (95% formamide, 10 mM EDTA, 0.01% xylene cyanol blue, 0.01% bromophenol blue) and heating at 70°C for 5 min. Reaction products were resolved in a 20% polyacrylamide gel containing 8 M urea and were visualized by staining with SYBR® Gold. Lanes: 1, LMW DNA size standards (nucleotides) (97, 77, 50, 40, 35, 30, 25, 20, 15); 2, no CIRCLIGASE negative control; 3, CIRCLIGASE from old clone; 4, CIRCLIGASE from new clone (lot 1); 5, CIRCLIGASE from new clone (lot 2); and 6, LMW DNA size standards.

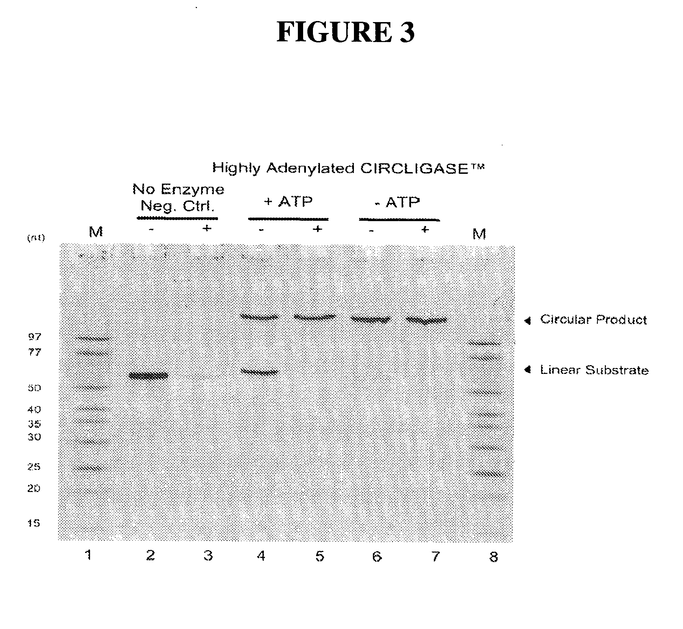

FIG. 3 shows a PAGE analysis of intramolecular ligation activities of the highly adenylated TS2126 RNA ligase (CIRCLIGASE™) in the presence and absence of ATP in the ligation reaction mixture. Surprisingly, the highly adenylated enzyme was much more active when ATP was not used in the ligation reaction mixture. Exonuclease I digests linear ssDNA, but does not digest circular ssDNA. After incubation for 1 hour at 60°C, the reactions were stopped and, where indicated, the reactions were incubated with 20U of Exo I at 37°C for 15 min. Reactions were stopped by addition of Stop/Load Buffer and heating at 70°C for 5 min. Reaction products were resolved in a 20% PAGE gel containing 8 M urea and stained with SYBR® Gold. Lanes: 1, LMW DNA size standards (97, 77, 50, 40, 35, 30, 25, 20, 15 nucleotides); 2 - 3, no enzyme controls; 4 - 5, Highly adenylated CIRCLIGASE ssDNA Ligase with 50 µM ATP; 6 - 7, Highly adenylated CIRCLIGASE ssDNA Ligase without ATP; 8, LMW DNA size standards.

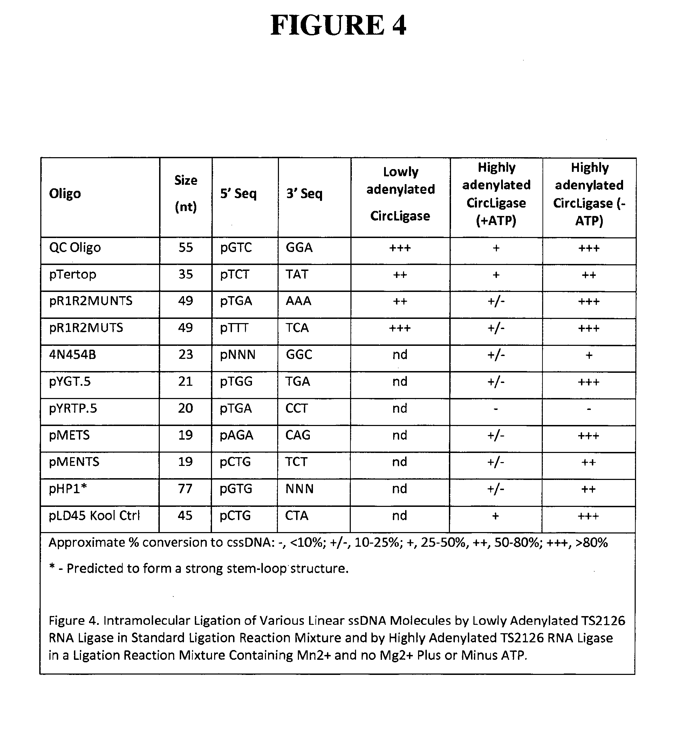

FIG. 4 is a Table showing the results of a PAGE analysis of the intramolecular ligation activity of the highly adenylated TS2126 RNA ligase (CIRCLIGASE™) from the new clone for a variety of linear ssDNA substrates, both in the presence and in the absence of ATP in the ligation reaction mixture, compared to intramolecular ligation activity of the lowly adenylated enzyme from the old clone in the Standard Ligation Reaction Mixture for those same linear ssDNA substrates. The results in show that, in the absence of ATP in the ligation reaction mixture, the highly adenylated TS2126 RNA ligase efficiently circularized many of the linear ssDNA substrates that were either not ligated or were inefficiently ligated by the lowly adenylated enzyme in the Standard Ligation Reaction Mixture. These improved intramolecular ligation results with the higly adenylated enzyme showed that certain substrates, which had previously been intramolecularly ligated poorly or not at all, could be ligated, which encouraged the applicants to continue to test additional ligation reaction conditions in order to improve the results still further.

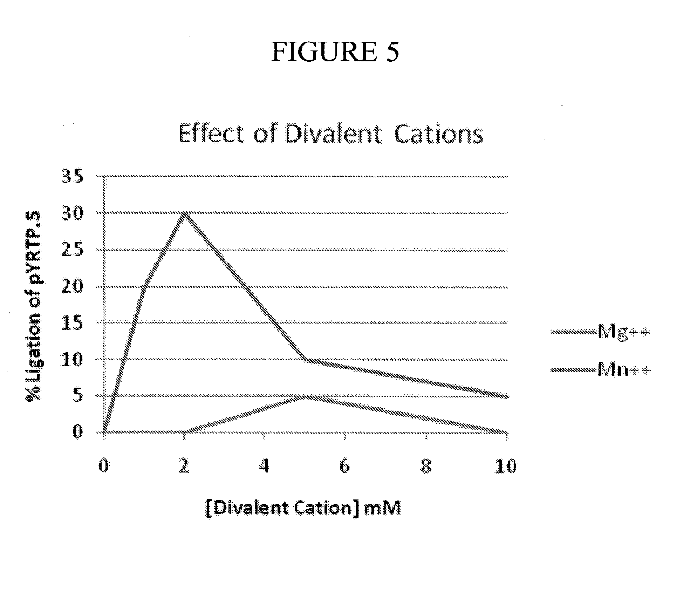

FIG. 5 shows that use of Mn²⁺ cations in the ligation reaction mixture improved the intramolecular ligation activity of a very difficult-to-ligate linear ssDNA substrate by the highly adenylated TS2126 RNA ligase (CIRCLIGASE™) in the absence of ATP. Note that Mg²⁺ cations, which are used in the Standard Ligation Reaction Mixture, were not added to the ligation reaction mixture when the Mn²⁺ cations were added. One µg of highly adenylated CIRCLIGASE™ ssDNA Ligase was incubated with 10 pmole of the linear pYRTP.5 ssDNA substrate in 33 mM Tris-acetate pH 7.6, 66 mM KOAc, 0.5 mM DTT, and 0, 1, 2, 5, or 10 mM MnCl2 or Mg(OAc)2 for 1 hour at 60°C. Then, a portion of the ligation reaction mixture was treated with 18U of Exo I and 20U of Exo III to degrade unligated linear substrate. Circular ssDNA ligation products were resolved in a 20% polyacrylamide gel containing 8 M urea and were visualized by staining with SYBR® Gold. The amount of exonuclease resistant circular ssDNA ligation product was estimated by visual inspection.

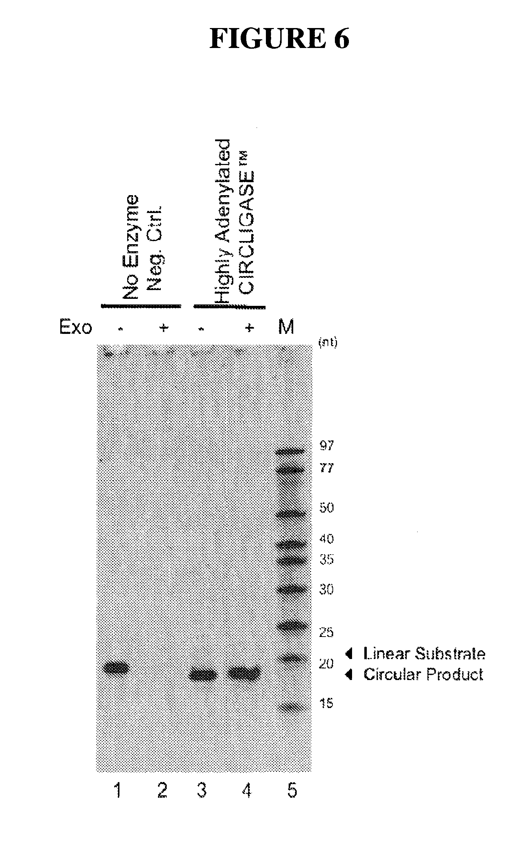

FIG. 6 shows a PAGE analysis of the intramolecular ligation activity of the highly adenylated TS2126 RNA ligase (CIRCLIGASE™) in circularizing a very difficult-to-ligate linear ssDNA substrate when 1M of zwitterionic trimethylglycine (betaine) was added to a ligation reaction mixture that that contained Mn²⁺ cations (as 2.5 mM MnCl₂), but lacked ATP and added Mg²⁺ cations. Surprisingly and unexpectedly, addition of betaine to the ligation reaction mixture resulted in approximately 100% circularization of this very difficult-to-ligate linear ssDNA substrate, compared to ≤ 5% circularization of this substrate by the lowly adenylated enzyme in the Standard Ligation Reaction Mixture. One µg of highly adenylated CIRCLIGASE™ ssDNA Ligase was incubated with 10 pmole of the linear pYRTP.5 ssDNA substrate in 33 mM Tris-acetate pH 7.6, 66 mM KOAc, 0.5 mM DTT, and 2.5 mM MnCl2, either with or without 1 M betaine, for 16 hours at 60°C. Then, a portion of the ligation reaction mixture was treated with 18U of Exo I and 20U of Exo III to degrade unligated linear substrate (lanes 1 and 4). Circular ssDNA ligation products were resolved in a 20% polyacrylamide gel containing 8 M urea and were visualized by staining with SYBR® Gold. The amount of exonuclease resistant circular ssDNA ligation product was estimated by visual inspection. Lanes: 1 - 2, No enzyme negative controls; 3 - 4, Plus 1 M betaine; 5, LMW DNA size standards.

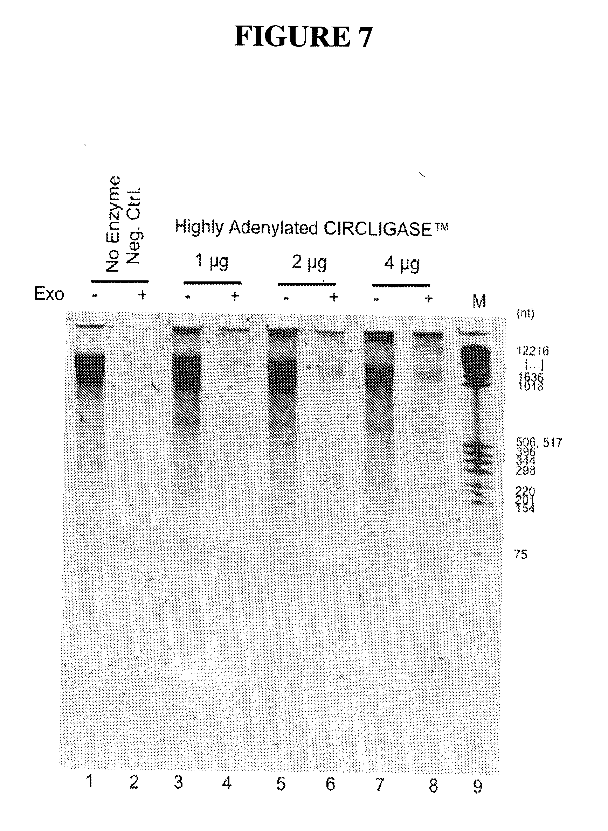

FIG. 7 shows a PAGE analysis of the intramolecular ligation activity of the highly adenylated TS2126 RNA ligase (CIRCLIGASE™) in circularizing Alu I-digested calf thymus ssDNA fragments in an Improved Ligation Reaction Mixture that contained 1 M betaine. The results indicated that there does not appear to be a gross substrate bias based on size or sequence composition for linear ssDNA fragments up to about 6000 nucleotides. Two hundred ng of denatured Alu I-digested calf thymus DNA in an improved ligation reaction mixture consisting of 33 mM Tris-acetate pH 7.6, 66 mM KOAc, 0.5 mM DTT, 2.5 mM MnCl2, 1 M betaine and 1, 2 or 4 µg of highly adenylated CIRCLIGASE™ ssDNA Ligase for 16 hours at 60°C and treated with 18U of Exo I and 20U of Exo III to degrade linear ssDNA substrate. Reaction products were resolved in a 20% polyacrylamide gel containing 8 M urea and were visualized by staining with SYBR® Gold. Lanes: 1, LMW DNA size standards; 2 - 3, No CIRCLIGASE negative controls; 4 - 5, 1 µg CIRCLIGASE; 6 - 7, 2 µg CIRCLIGASE; 8 - 9, 4 µg CIRCLIGASE; 10, Kb Ladder DNA size standards. Exonucleases were added to lanes 3, 5, 7, and 9. It should be noted that CIRCLIGASE is expected to function stoichiometrically in the absence of ATP; therefore it is not surprising that only 10% of the 200 ng input of denatured fragmented ssDNA was circularized.

### DEFINITIONS

Unless specifically defined or described differently elsewhere herein, the following terms and descriptions related to the invention shall be understood as given below.

When the terms "for example", "e.g.", "such as", "include", "including" or variations thereof are used herein, these terms will not be deemed to be terms of limitation, and will be interpreted to mean "but not limited to" or "without limitation."

The use of terms "a" and "an" and "the" and similar referents in the context of describing the invention (especially in the context of the claims) are to be construed to cover both the singular and the plural, unless otherwise indicated herein or clearly contradicted by context.

"Affinity binding substances" or "affinity binding molecules" or "affinity molecules" or "affinity tags" herein means molecules that have affinity for and "bind" to each other under certain conditions, referred to as "binding conditions", to form a "specific binding pair." For example, biotin and streptavidin, biotin and avidin, or digoxigenin and a specific antibody that binds digoxigenin are examples of "specific binding pairs," with the members of each specific binding pair comprising "affinity binding molecules" or "affinity binding substances" or "affinity molecules." Affinity binding molecules (e.g., biotin and / or streptavidin) can be covalently joined or conjugated, or non-covalently bound, to other molecules (e.g., to RNA or DNA) or to a solid surface using methods known in the art (e.g., using reagents and methods as described in Avidin-Biotin Chemistry: A Handbook, by D. Savage et al., Pierce Chemical Company, 1992, and in Handbook of Fluorescent Probes and Research Products, Ninth Edition, by R.P. Hoagland, Molecular Probes, Inc., and in BIOCONJUGATE Techniques, by Greg T. Hermanson, Published by Academic Press, Inc., San Diego, CA, 1996). Affinity molecules that are conjugated to DNA or RNA can also be synthesized using an oligonucleotide synthesizer using reagents and methods known in the art.

The term "binding" according to the present invention means the interaction between an affinity molecule and an affinity binding substance as a result of non-covalent bonds, such as, but not limited to, hydrogen bonds, hydrophobic interactions, van der Waals bonds, and ionic bonds. Without being bound by theory, it is believed in the art that these kinds of non-covalent bonds result in binding, in part due to complementary shapes or structures of the molecules involved in the specific binding pair. Based on the definition for "binding," and the wide variety of affinity binding molecules or specific binding pairs, it is clear that binding conditions vary for different specific binding pairs. Those skilled in the art can easily find or determine conditions whereby, in a sample, binding occurs between the affinity binding molecules. In particular, those skilled in the art can easily determine conditions whereby binding between affinity binding molecules that would be considered in the art to be "specific binding" can be made to occur. As understood in the art, such specificity is usually due to the higher affinity between the affinity binding molecules than for other substances and components (e.g., vessel walls, solid supports) in a sample. In certain cases, the specificity might also involve, or might be due to, a significantly more rapid association of affinity binding molecules than with other substances and components in a sample.

The term "amplifying a nucleic acid" herein means increasing the number of copies of a nucleic acid sequence or its complement. The nucleic acid that is amplified can be DNA comprising, consisting of, or derived from DNA or RNA or a mixture of DNA and RNA, including modified DNA and/or RNA. The products resulting from amplification of a nucleic acid molecule or molecules (i.e., "amplification products"), whether the starting nucleic acid is DNA, RNA or both, can be either DNA or RNA, or a mixture of both DNA and RNA nucleosides or nucleotides, or they can comprise modified DNA or RNA nucleosides or nucleotides. A "copy" does not necessarily mean perfect sequence complementarity or identity to the target sequence. For example, copies can include nucleotide analogs such as deoxyinosine or deoxyuridine, intentional sequence alterations (such as sequence alterations introduced through a primer comprising a sequence that is hybridizable, but not complementary, to the target sequence, and/or sequence errors that occur during amplification.

As used herein, the terms "amplify" or "amplified" "amplifying" as used in reference to a nucleic acid or nucleic acid reactions, refer to *in vitro* methods of making copies of a particular nucleic acid, such as a target nucleic acid, or a tagged nucleic acid produced, for example, by an embodiment of the present invention. Numberous methods of amplifying nucleic acids are known in the art, and amplification reactions include polymerase chain reactions, ligase chain reactions, strand displacement amplification reactions, rolling circle amplification reactions, transcription-mediated amplification methods such as NASBA (e.g., U.S. Pat. No. 5,409,818), loop mediated amplification methods (e.g., "LAMP" amplification using loop-forming sequences, e.g., as described in U.S. Patent No. 6,410,278).

The terms "anneal" or "hybridize" and "annealing" or "hybridization" refer to the formation of complexes between nucleotide sequences that are sufficiently complementary to form complexes via Watson-Crick base pairing. With respect to the present invention, nucleic acid sequences that are "complementary to" or "complementary with" or that "hybridize" or "anneal" to or with each other should be capable of forming or form "hybrids" or "complexes" that are sufficiently stable to serve the intended purpose. Hybridization or annealing and the strength of hybridization (i.e., the strength of the association between nucleic acid strands) is impacted by many factors well known in the art including the degree of complementarity between the nucleic acids, stringency of the conditions involved affected by such conditions as the concentration of salts, the Tₘ (melting temperature) of the formed hybrid, the presence of other components (e.g., the presence or absence of polyethylene glycol or betaine), the molarity of the hybridizing strands and the G:C content of the nucleic acid strands.

In some embodiments, "cDNA" or a "cDNA molecule" refers to "complementary DNA" that is synthesized by RNA-dependent DNA polymerase- or reverse transcriptase-catalyzed extension of a primer that anneals to one or more RNA molecules of interest using at least a portion of the RNA molecules of interest as templates (which process is also called "reverse transcription"). In some preferred embodiments of the method, the first-strand cDNA molecules are synthesized by reverse transcription using a reverse transcriptase and RNA molecules of interest, such as messenger RNA (mRNA) molecules, obtained from a biological sample as a template, and which first-strand cDNA molecules are complementary the mRNA. In some embodiments, the "first-strand cDNA molecules" refer to cDNA molecules synthesized by reverse transcription of any RNA molecule of interest, even if it is not mRNA. In some embodiments, the terms "first-strand cDNA synthesis primer" and "first-strand cDNA molecules" are used even if no second-strand cDNA synthesis primer is used and no second-strand cDNA molecules are synthesized; thus, the terms "first-strand cDNA" or "first-strand cDNA molecules" are used even when the method results in synthesis of only single-stranded cDNA that is complementary to the RNA molecules of interest. Still further, in some embodiments, the term "cDNA" herein refers to complementary DNA that is synthesized by a DNA polymerase-catalyzed extension of a primer that anneals to one or more DNA molecules of interest using at least a portion of the DNA molecule of interest as templates. The cDNA molecules are "homologous to" or "base pair with" or "form a complex with" at least a portion of the template.

As used herein, a "DNA polymerase" refers to an enzyme that catalyzes the polymerization of deoxyribonucleotides into a DNA strand. A "DNA-dependent DNA polymerase" is an enzyme that synthesizes a complementary DNA ("cDNA") copy by extension of a primer that is annealed to a DNA template. Some DNA-dependent DNA polymerases may also synthesize a complementary DNA copy from an RNA template, a process that is also referred to as "reverse transcription." DNA polymerases that can reverse-transcribe can also be referred to as a "reverse transcriptases."

In addition to synthesizing DNA polymers, DNA polymerases may comprise other features or activities. For example, a DNA polymerase may have or lack 5' to 3' exonuclease activity (also referred to a 5' exonuclease or 5' nuclease activity), 3' to 5' exonuclease activity, strand displacement activity, and they may be characterized with respect to the degree they are processive. In some embodiments, a DNA polymerase is used that lacks 5'-to-3' exonuclease activity. For example, in some embodiments, a DNA polymerase composition that lacks 5'-to-3' exonuclease activity is used for DNA sequencing. For example, in some other embodiments, a DNA polymerase composition that lacks 5'-to-3' exonuclease activity is used for whole genome amplification.

Some DNA polymerases are able to displace the strand complementary to the template strand as a new DNA strand is synthesized by the polymerase. This process is called "strand displacement" and the DNA polymerases that have this activity are referred to herein as "strand-displacing DNA polymerases." The template for strand displacement DNA synthesis can be a linear or circular single-stranded DNA (ssDNA) or double-stranded DNA (dsDNA). If the DNA template is a single-stranded circle, primed DNA synthesis procedes around and around the circle, with continual displacement of the strand ahead of the replicating strand, a process called "rolling circle replication." Rolling circle replication results in synthesis of tandem copies of the circular template. In general, it is preferred that a DNA-template-specific DNA polymerase used for a method of the invention efficiently synthesizes DNA of a suitable length for the intended purpose without "falling off" of the template (or terminating synthesis of the DNA), which is referred to as the enzyme's processivity. The capability of a DNA polymerase to strand displace can be readily determined using the polymerase in a rolling circle replication assay as described by Fire and Xu (Proc. Natl. Acad. Sci. USA 92: 4641-4645, 1995). Strand displacement and DNA polymerase processivity can also be assayed using methods described in Kong et al. (J. Biol. Chem. 268: 1965-1975, 1993).

Examples of strand-displacing DNA polymerases that can be used include, but are not limited to, RepliPHI™ phi29 DNA polymerase, DisplaceAce™ DNA polymerase, rGka DNA polymerase, SequiTherm™ DNA polymerase, Taq DNA polymerase, Tfl DNA polymerase, and MMLV reverse transcriptase (all available from EPICENTRE Biotechnologies, Madison, WI, USA). In some embodiments, a blend of a DNA polymerase that lacks 3'-to-5' exonuclease proofreading activity with a DNA polymerase that has this activity, such as FAILSAFE™ DNA polymerase is used as the strand-displacing DNA polymerase. The enzyme blend is useful in some embodiments because it exhibits improved fidelity during DNA synthesis (i.e., it synthesizes DNA with fewer nucleotides that are not complementary to the template). Fidelity and/or error rates of many DNA polymerases under particular conditions are known, as are methods for measuring fidelity (e.g., by sequencing).

In general, it is desirable in a strand-displacement amplification method of the present invention that the amount of strand-displacing DNA polymerase used in the method is as high as possible without inhibiting or adversely affecting the reaction. For example, REPLIPHI™ phi29 DNA polymerase (EPICENTRE) can be used at about one microgram of protein in a 20-microliter reaction and DISPLACE™ DNA polymerase (EPICENTRE) can be used at about 50 units to about 300 units in a 50-microliter reaction. Since definitions for units vary for different DNA polymerases and even for similar DNA polymerases from different vendors or sources, and also because the activity for each enzyme varies at different temperatures and under different reaction conditions, it is desirable to optimize the amount of strand-displacing DNA polymerase and reaction conditions for each DNA template and primer used.

A "nucleic acid" or "polynucleotide" means a polymer molecule comprising a series of "mononucleosides," also referred to as "nucleosides," in which the 3'-position of the pentose sugar of one nucleoside is linked by an internucleoside linkage, such as, but not limited to, a phosphodiester bond, to the 5'-position of the pentose sugar of the next nucleoside. A nucleoside linked to a phosphate group is referred to as a "nucleotide." The nucleotide that is linked to the 5'-position of the next nucleotide in the series is referred to as "5' of" or the "5' nucleotide" and the nucleotide that is linked to the 3'-position of the 5' nucleotide is referred to as "3' of" or the "3' nucleotide." As used herein, the terms "5'-of" and "3'-of" refer to the position or orientation of a particular chemical group, nucleotide, sequence of nucleotides, or genetic element (e.g., an RNA polymerase promoter sequence) relative to another chemical group, nucleotide, sequence of nucleotides, or genetic element within a single strand of a nucleic acid. If a first nucleic acid sequence is 3'-of a second sequence on one strand, the complement of the first sequence will be 5'-of the complement of the second sequence on the complementary strand. The description of the invention will be understood with respect to the relative 5' or 3' position and orientation of a sequence or genetic element within a particular nucleic acid strand.

Linear nucleic acid molecules are said to have a "5'-terminus" (5' end) and a "3'-terminus" (3' end) because nucleic acid phosphodiester linkages occur at the 5' carbon and 3' carbon of the sugar moieties of the substituent mononucleotides. The end of a polynucleotide at which a new linkage would be to a 5' carbon is its 5' terminal nucleotide. The end of a polynucleotide at which a new linkage would be to a 3' carbon is its 3' terminal nucleotide. A terminal nucleotide, as used herein, is the nucleotide at the end position of the 3'- or 5'-terminus.

The pentose sugar of the nucleic acid can be ribose, in which case, the nucleic acid or polynucleotide is referred to as "RNA," or it can be 2'-deoxyribose, in which case, the nucleic acid or polynucleotide is referred to as "DNA." Alternatively, especially if the nucleic acid is synthesized chemically, the nucleic acid can be composed of both DNA and RNA mononucleotides. In both RNA and DNA, each pentose sugar is covalently linked to one of four common or "canonical" nucleic acid bases (each also referred to as a "base"). Three of the predominant naturally-occurring bases that are linked to the sugars (adenine, cytidine and guanine) are common for both DNA and RNA, while one base is different; DNA has the additional base thymine, while RNA has the additional base uridine. In some cases, uridine can be present as a base in DNA. Those in the art commonly think of a small polynucleotide as an "oligonucleotide." The term "oligonucleotide" as used herein is defined as a molecule comprising of two or more deoxyribonucleotides (in which case, it may also be referred to as an "oligodeoxyribonucleotide." or ribonucleotides, preferably about 6 to 100 nucleotides, but there is no defined limit to the length of an oligonucleotide. The exact size will depend on many factors, which in turn depends on the ultimate function or use of the oligonucleotide.

Also, for a variety of reasons, a nucleic acid or polynucleotide of the invention may comprise one or more modified nucleic acid bases, sugar moieties, or internucleoside linkages. By way of example, some reasons for using nucleic acids or polynucleotides that contain modified bases, sugar moieties, or internucleoside linkages include: (1) modification of the Tₘ; (2) changing the susceptibility of the polynucleotide to one or more nucleases; (3) providing a cleavage site, such as a dUMP residue, which is cleaved by uracil-N-glycosylase plus alkaline conditions or an endonuclease, e.g., endonuclease IV, or, such as an 8-oxo-dGMP residue, which is cleaved by 8-oxoguanine DNA glycosylase (also known as [fapy-DNA glycosylase or Fpg) plus alkaline conditions or an endonuclease, e.g., endonuclease IV; (4) providing a moiety for attachment of a label or an affinity tag; (5) providing a label or a quencher for a label; or (6) providing a moiety, such as biotin, as an affinity tag for attaching to another molecule which is in solution or bound to a surface.

With respect to nucleic acids or polynucleotides of the invention, one or more of the sugar moieties can comprise 2'-deoxyribose, or alternatively, one or more of the sugar moieties can be some other sugar moiety, such as, but not limited to, ribose, or 2'-fluoro-2'-deoxyribose or 2'-O-methyl-ribose, which provide resistance to some nucleases, or 2'-amino-2'-deoxyribose or 2'-azido-2'-deoxyribose, which can be labeled by reacting them with visible, fluorescent, infrared fluorescent or other detectable dyes or chemicals having an electrophilic, photoreactive, alkynyl, or other reactive chemical moiety.

The internucleoside linkages of nucleic acids or polynucleotides of the invention can be phosphodiester linkages, or alternatively, one or more of the internucleoside linkages can comprise modified linkages, such as, but not limited to, phosphorothioate, phosphorodithioate, phosphoroselenate, or phosphorodiselenate linkages, which are resistant to some nucleases.

When referring to an oligonucleotide or a portion of an oligonucleotide that exhibits a "random sequence", we mean that the oligonucleotide or portion thereof is synthesized (e.g., using an oligonucleotide synthesizer) using equal amounts of all four of the canonical nucleotide bases (A, G, C, and T or U) for very nucleotide position within the random sequence portion. This method results in synthesis of a mixture of oligonucleotides comprising (4 to the n power) + 1 of different oligonucleotides, where "n" equals the number of nucleotide positions within the random sequence portion. Thus, in these embodiments, the oligonucleotide comprises a mixture of many different oligonucleotides, representing all possible sequences for the random sequence portion. When referring to an oligonucleotide or a portion of an oligonucleotide that exhibits a "semi-random sequence", we mean that the semi-random oligonucleotide or portion is synthesized (e.g., using an oligonucleotide synthesizer) wherein some nucleotide postions are synthesized using equal amounts of all four of the canonical nucleotide bases (A, G, C, and T or U) (i.e., those positions are "random" as described above) but one or more other positions within the semi-random portion are synthesized using only one, two, or three, rather than all four, of the canonical base nucleotides (i.e., A, C, G, and T or U). In some embodiments, an oligonucleotide contains one or more nucleotides with a "degenerate base", by which we mean a nucleic acid base that is capable of base-pairing with one or more nucleic acid bases other than according to the standard base-pairing rules that A pairs with T or U and G pairs with C, and a "degenerate nucleotide" is a nucleotide that contains a degenerate base. A "portion" or "region," used interchangeably herein, of a polynucleotide or oligonucleotide (including a primer) is a contiguous sequence of 2 or more bases. In other embodiments, a region or portion is at least about any of 1, 2, 3, 5, 10, 15, 20, 25, 50, 75, or even more contiguous nucleotides. If the random or semi-random sequence comprises all of the nucleotides in the oligonucleotide, it may be referred to, respectively, as a "random oligonucleotide" or a "semi-random oligonucleotide."

A "primer" is an oligonucleotide ("oligo"), generally with a free 3'-OH group, that can be extended by a nucleic acid polymerase. For a template-dependent polymerase, generally at least the 3'-portion of the primer oligo is complementary to a portion of a template nucleic acid, to which the oligo "binds" (or "complexes," "anneals," or "hybridizes"), by hydrogen bonding and other molecular forces, to the template to give a primer/template complex for initiation of synthesis by a DNA polymerase, and which is extended (i.e., "primer extended") by the addition of covalently bonded bases linked at its 3'-end which are complementary to the template in the process of DNA synthesis. The result is a primer extension product. Template-dependent DNA polymerases (including reverse transcriptases) generally require complexing of an oligonucleotide primer to a single-stranded template to initiate DNA synthesis ("priming"), but RNA polymerases generally do not require a primer for synthesis of RNA that is complementary to a DNA template (transcription).

A "thermostable RNA ligase" herein means a polypeptide, wherein the adenylated form of said polypeptide catalyzes template-independent circularization of linear ssDNA molecules that have 5'-phosphoryl and 3'-hydroxyl groups to circular ssDNA molecules at a reaction temperature between about 40 degrees C and about 70 degrees C in a reaction mixture that comprises a buffer that maintains the pH at between about pH 6.5 and pH 8, Mn²⁺ cations at a concentration between about 0.5 and 10 mM, and wherein ATP is either not present in the reaction buffer or is present at a molar concentration that is less than the concentration of the non-adenylated form of the polypeptide. A preferred thermostable RNA ligase of the present invention is bacteriophage TS2126 thermostable RNA ligase, as described and covered by the claims in U.S. Patent No. 7,303,901. However, the thermostable RNA ligase can comprise any RNA ligase that is active in synthesizing circular ssDNA molecules from linear ssDNA molecules in the ligation reaction mixtures and under the reaction conditions described herein.

The thermostable RNA ligase can be from a native protein or a recombinant protein. The term "native protein" is used herein to indicate a protein isolated from a naturally occurring (i.e., a nonrecombinant) source. The term "recombinant protein" or "recombinant polypeptide" as used herein refers to a protein molecule expressed from a recombinant DNA molecule. Molecular biological techniques may be used to produce a recombinant form of a protein with identical or similar properties as compared to the native form of the protein. Variants of the native sequence may also be made to, for example, improve expression, purification, or other desired properties of the polypeptide.

The thermostable RNA ligase that is a recombinant protein can be a fusion protein. As used herein, the term "fusion protein" refers to a chimeric protein containing the protein of interest (e.g., the TS2126 RNA ligase or fragments thereof) joined to an exogenous protein fragment (e.g., the fusion partner which contains a non-TS2126 RNA ligase protein). The fusion partner may enhance the solubility of the thermostable RNA ligase protein as expressed in a host cell, may provide an affinity tag to allow purification of the recombinant fusion protein from the host cell or culture supernatant, or both. If desired, the fusion protein may be removed from the protein of interest (e.g., TS2126 RNA ligase or fragments thereof) by a variety of enzymatic or chemical means known to the art.

In preferred embodiments of the present invention, the thermostable RNA ligase composition comprises a purified protein. As used herein, the term "purified" or "to purify" means the result of any process that removes some of a contaminant from the component of interest, such as a protein. For example, a particular desired protein (e.g., TS2126 RNA ligase) is purified by removal of other contaminating undesired proteins, nucleic acid, carbohydrate, lipid and/or small biochemical molecules. The removal of contaminants results in an increase in the percentage of desired protein in the composition. For example, in preferred embodiments, the thermostable RNA ligase composition is purified so as to be free of contaminating nucleic acids and enzymes with activity on nucleic acids.

In some preferred embodiments, the thermostable RNA ligase is obtained by expression of the thermostable RNA ligase gene (and/or functional variants and homologues thereof) in a plasmid or other vector that is replicated and expressed in *Escherichia coli* cells, since the thermostable RNA ligase obtained from such a recombinant source is of a higher purity, free from contaminating enzymatic activities, and generally at a higher enzyme concentration than is obtained from nonrecombinant sources. The term "gene" as used herein, refers to a DNA sequence that comprises control and coding sequences necessary for the production of the encoded polypeptide or protein precursor (e.g., TS2126 RNA ligase). The polypeptide can be encoded by a full-length coding sequence or by any portion of the coding sequence, as long as the desired protein activity is retained.

In preferred embodiments of the invention, the thermostable RNA ligase is "stabilized", by which we mean that the thermostable RNA ligase is sufficiently pure of proteases and other contaminants which contribute to degradation and loss of enzyme activity and is provided in a formulation of enzyme storage buffer in which there is no significant loss of activity during storage at -20 degrees C for at least six months. One suitable enzyme storage buffer for providing a stabilized thermostable RNA ligase (e.g., TS2126 RNA ligase) comprises a 50% glycerol solution containing 50 mM Tris-HCl (pH 7.5), 100 mM NaCl, 100 mM EDTA, 1 mM DTT and 0.1% of the non-ionic detergent Triton X-100. The term "thermostable RNA ligase", as used herein, can refer to the variants of the protein or to the gene, unless indicated otherwise.

Moreover, variant forms of the thermostable RNA ligase are also contemplated as being equivalent to those peptides and DNA molecules that are set forth in more detail herein. For example, it is contemplated that isolated replacement of a leucine with an isoleucine or valine, an aspartate with a glutamate, a threonine with a serine, or a similar replacement of an amino acid with a structurally related amino acid (*i.e.,* conservative mutations) will not have a major effect on the biological activity of the resulting molecule. Accordingly, some embodiments of the present invention provide variants of the thermostable RNA ligase (e.g., TS2126 RNA ligase) contain conservative replacements. Conservative replacements are those that take place within a family of amino acids that are related in their side chains. Genetically encoded amino acids can be divided into four families: (1) acidic (aspartate, glutamate); (2) basic (lysine, arginine, histidine); (3) nonpolar (alanine, valine, leucine, isoleucine, proline, phenylalanine, methionine, tryptophan); and (4) uncharged polar (glycine, asparagine, glutamine, cysteine, serine, threonine, tyrosine). Phenylalanine, tryptophan, and tyrosine are sometimes classified jointly as aromatic amino acids. In similar fashion, the amino acid repertoire can be grouped as (1) acidic (aspartate, glutamate); (2) basic (lysine, arginine, histidine), (3) aliphatic (glycine, alanine, valine, leucine, isoleucine, serine, threonine), with serine and threonine optionally be grouped separately as aliphatic-hydroxyl; (4) aromatic (phenylalanine, tyrosine, tryptophan); (5) amide (asparagine, glutamine); and (6) sulfur -containing (cysteine and methionine) (*e.g*., Stryer ed., Biochemistry, pg. 17-21, 2nd ed, WH Freeman and Co., 1981). It can be readily determined whether a change in the amino acid sequence of a peptide results in a functional polypeptide by assessing the ability of the variant peptide to function in a fashion similar to the wild-type protein. Peptides having more than one replacement can readily be tested in the same manner. More rarely, a variant includes "nonconservative" changes (*e.g.,* replacement of a glycine with a tryptophan). Analogous minor variations can also include amino acid deletions or insertions, or both. Guidance in determining which amino acid residues can be substituted, inserted, or deleted without abolishing biological activity can be found using computer programs (*e.g*., LASERGENE software, DNASTAR Inc., Madison, WI).

Variants may be produced by methods such as directed evolution or other techniques for producing combinatorial libraries of variants, as well as truncation mutants. In some embodiments, homologs and variants are generated from a degenerate oligonucleotide sequence, e.g., made by chemical synthesis of a degenerate gene sequence in an automatic DNA synthesizer, and then ligated to assemble an appropriate gene for expression. In some embodiments, artificial evolution is performed by random mutagenesis (*e.g*., by utilizing error-prone PCR to introduce random mutations into a given coding sequence). After mutagenesis, the resulting clones are selected for desirable activity (*e.g*., screened for thermostable RNA ligase activity), and then useful mutations are carried over to the next round of mutagenesis, in a process of successive rounds of mutagenesis and selection. In other embodiments of the present invention, the polynucleotides of the present invention are used in gene shuffling or sexual PCR procedures (e.g., Smith, Nature, 370: 324, 1994; U.S. Pat. Nos. 5837458; 5830721; 5811238; 5733731). Multiple cycles of selection and shuffling have led to the functional enhancement of several enzymes (Stemmer, Nature, 370:398, 1994; Stemmer, Proc. Natl. Acad. Sci. USA, 91: 10747, 1994; Crameri et al., Nat. Biotech., 14: 315, 1996; Zhang et al., Proc. Natl. Acad. Sci. USA, 94: 4504, 1997; and Crameri et al., Nat. Biotech., 15: 436, 1997). Fragments of the nucleic acids and proteins of the present invention may also be used, so long as the fragments encode or possess the desired enzymatic activity.

A "single-strand-specific DNase" means a DNase that specifically digests single-stranded DNA, but that does not digest single-stranded RNA or RNA or DNA that is annealed to or complexed with complementary RNA or DNA, whether said complementary RNA or DNA is part of another nucleic acid molecule (e.g., by intermolecular base-pairing) or a portion of the same nucleic acid molecule (e.g., by intramolecular base-pairing). The single-strand-specific DNase can be an endonuclease or an exonuclease, so long as it is active in specifically digesting single-stranded DNA to monomers or short oligodeoxyribonucleotides. In some preferred embodiments, oligodeoxyribonucleotides, including primers, are removed from the reaction mixture after step of the method in which they are used by digestion with a single-strand-specific DNase. Exonuclease I, exonuclease VII, and Rec J exonuclease are exemplary single-strand-specific DNases.

A "T7-type RNA polymerase" (RNAP) herein means T7 RNA polymerase (e.g., see Studier, FW et al., pp. 60-89 in Methods in Enzymology, Vol. 185, ed. by Goeddel, DV, Academic Press, 1990) or an RNAP derived from a "T7-type" bacteriophage, meaning a bacteriophage that has a similar genetic organization to that of bacteriophage T7. In some embodiments, an RNA polymerase promoter can be single-stranded, such as a pseudopromoter (e.g., Ohmichi et al., Proc. Natl. Acad. Sci. USA 99:54-59, 2002), or an N4 vRNAP promoter, in which case the truncated protein comprising the transcriptionally active 1,106-amino acid domain (corresponding to amino acids 998-2103) of the N4 vRNAP (designated "mini-vRNAP"; EPICENTRE Biotechnologies, Madison, WI, USA) is used (Kazmierczak, K.M., et al., EMBO J., 21: 5815-5823, 2002).
As used herein, a "DNA fragment" means a portion or piece or segment of a longer DNA molecule that is cleaved from or released or is broken from the longer DNA molecule such that it is no longer attached to the parent molecule, or a ssDNA molecule that is a complementary copy of only a portion of the longer DNA molecule, in which case, the complementary copy is synthesized by using a DNA polymerase to extend a primer that anneals to and uses the longer DNA molecule as a template. In some preferred embodiments, the method is used to generate a "DNA fragment library" comprising a collection or population of tagged DNA fragments.

A "template" is a nucleic acid molecule that is being copied by a nucleic acid polymerase, such as a DNA polymerase. Whether the nucleic acid molecule comprises two strands (i.e., is "double-stranded") or only one strand (i.e., is "single-stranded"), the strand of said nucleic acid molecule that serves to specify the sequence of nucleotides exhibited by a nucleic acid that is synthesized is the "template" or "the template strand." The nucleic acid synthesized by the nucleic acid polymerase is complementary to the template. Both RNA and DNA are always synthesized in the 5'-to-3' direction, beginning at the 3'-end of the template strand, and the two strands of a nucleic acid duplex always are aligned so that the 5' ends of the two strands are at opposite ends of the duplex (and, by necessity, so then are the 3' ends). A primer is required for both RNA and DNA templates to initiate synthesis by a DNA polymerase, but a primer is not required to initiate synthesis by a DNA-dependent RNA polymerase, which is usually called simply an "RNA polymerase."

As used herein, a "tag" refers to a non-target nucleic acid component, generally DNA, which provides a means of addressing a nucleic acid fragment to which it is joined. For example, in preferred embodiments, a tag comprises a nucleotide sequence that permits identification, recognition, and/or molecular or biochemical manipulation of the DNA to which the tag is attached (e.g., by providing a site for annealing an oligonucleotide, such as a primer for extension by a DNA polymerase, or an oligonucleotide for capture or for a ligation reaction). The process of joining the tag to the DNA molecule is sometimes referred to herein as "tagging" and DNA that undergoes tagging or that contains a tag is referred to as "tagged" (e.g., "tagged DNA")." The tag can have one or more tag portions or tag domains.

As used herein, a "tag portion" or a "tag domain" means a portion or domain of a tag that exhibits a sequence for a desired intended purpose or application. In embodiments wherein the first-strand cDNA synthesis primer exhibits one or more nucleotide sequences in its 5'-end portion that are not complementary to a target nucleic acid sequence, the tag has one or more "tag domains" in said 5'-portion, each of which tag domains is provided for any desired purpose. For example, in some embodiments, the tag comprises or consists of one or more tag domains selected from among a cleavage site tag domain, an RNA polymerase promoter tag domain; a sequencing tag domain, a capture tag domain, an amplification tag domain, a detection tag domain, an address tag domain, and a transposon end domain.

As used herein, a "cleavage site domain" means a tag domain that exhibits a sequence for the purpose of facilitating cleavage. In some embodiments, the cleavage site domain is used to generate di-tagged linear ssDNA molecules from tagged circular ssDNA molecules. In some embodiments, the cleavage site domain in the tag comprises or consists of one or more 2'-deoxyuridine monophosphate (dUMP) moieties or one or more 8-oxoguanine-2'-deoxyribosyl-monophosphate (8-oxo-dGMP) moieties and step (c) of the method comprises contacting the tagged circular first-strand cDNA molecules with uracil-N-glycosylase (UNG; EPICENTRE) or 8-oxoguanine DNA glycosylase (Fpg; EPICENTRE), respectively, to generate tagged circular first-strand cDNA molecules that contain one or more abasic sites, and then incubating the tagged circular first-strand cDNA molecules that contain the one or more abasic sites under conditions, such as by incubating in an alkaline solution or in a solution that contains an endonuclease such as endonuclease IV, wherein the circular first-strand cDNA molecules are linearized at or near the abasic sites to generate di-tagged linear ssDNA molecules. In some embodiments, the cleavage site domain in the tag exhibits the sequence of a restriction site. In some embodiments, the restriction site is present only rarely, if at all, in the target DNA (e.g., a restriction site for a rare-cutting restriction endonuclease such as NotI or AscI). In some embodiments, the restriction site in the cleavage site domain is for a type II restriction endonuclease, such as FokI restriction endonuclease. In some embodiments, the method further comprises: annealing an oligodeoxyribonucleotide that is complementary to the single-stranded restriction site of the tagged circular ssDNA fragments and then cleaving the tagged circular ssDNA fragments at the restriction site using the restriction endonuclease that recognizes the restriction site. Thus, in some embodiments, the method comprises linearizing the tagged circular ssDNA fragments to generate di-tagged linear ssDNA fragments. In some other embodiments, the first-strand cDNA synthesis primer has a 5'-end portion comprising or consisting of a double-stranded hairpin comprising the restriction site, and the method further comprises the steps of cleaving the tagged linear ssDNA fragments at the restriction site using the restriction endonuclease that recognizes the restriction site. In some preferred embodiments comprising (i) generating a double-stranded restriction site, either by annealing of an oligodeoxyribonucleotide that is complementary to the single-stranded restriction site, or by using a transferred strand that comprises a double-stranded hairpin, and (ii) then cleaving the restriction site using the restriction endonuclease that recognizes the double-stranded restriction site, the method further comprises the step of ligating the restriction endonuclease-cleaved tagged linear ssDNA fragments to another DNA molecule that has a compatible 3'-end.

As used herein, an "RNA polymerase promoter domain" or a "promoter domain" means a tag domain that exhibits a sequence for a sense promoter sequence or for an anti-sense promoter sequence of an RNA polymerase promoter. As used herein, a "sense promoter sequence" or a "sense RNA polymerase promoter sequence" means the sequence of an RNA polymerase promoter that is joined to the DNA strand that serves as the template for transcription by an RNA polymerase which binds the RNA polymerase promoter and initiates transcription therefrom under reaction conditions suitable for transcription. As used herein, an "anti-sense promoter sequence" or an "anti-sense RNA polymerase promoter sequence" means the sequence of an RNA polymerase promoter that is complementary to the sense promoter sequence. In some embodiments, the sense promoter sequence exhibited by the RNA polymerase promoter domain is for an RNA polymerase that binds a single-stranded RNA polymerase promoter and initiates transcription therefrom, in which embodiments the sense promoter sequence is sufficient to function as the RNA polymerase promoter (e.g., for bacteriophage N4 RNA polymerase). In some embodiments, the sense promoter sequence is for an RNA polymerase that binds a double-stranded RNA polymerase promoter and initiates transcription therefrom, in which embodiments the method comprises making the RNA polymerase promoter double-stranded (e.g., by annealing to the sense promoter sequence an oligodeoxyribonucleotide that exhibits an anti-sense promoter sequence that is complementary to the sense promoter sequence, or by using the tagged circular ssDNA fragments or the di-tagged linear ssDNA fragments as templates for synthesis of dsDNA comprising or consisting of the sense promoter sequence) prior to transcription using an RNA polymerase that binds to and initiates transcription from the double-stranded RNA polymerase promoter. In some embodiments, the sense promoter sequence is for a T7-type RNA polymerase (e.g., selected from among T7 RNA polymerase, T3 RNA polymerase, and SP6 RNA polymerase). A RNA polymerase promoter domain that exhibits a sense promoter sequence enables synthesis of RNA that is complementary to the single-stranded DNA to which the sense promoter sequence is ligated using the method.

As used herein, a "sequencing tag domain" or a "sequencing tag" means a tag domain that exhibits a sequence for the purposes of facilitating sequencing of the ssDNA fragment to which the tag is joined using the method to synthesize tagged circular ssDNA fragments (e.g., to provide a priming site for sequencing by synthesis, or to provide annealing sites for sequencing by ligation, or to provide annealing sites for sequencing by hybridization). For example, in some embodiments, the sequencing tag domain provides a site for priming DNA synthesis of said ssDNA fragment or the complement of said ssDNA fragment.

As used herein, a "capture tag domain" or a "capture tag" means a tag domain that exhibits a sequence for the purpose of facilitating capture of the ssDNA fragment to which the tag domain is joined (e.g., to provide an annealing site or an affinity tag for a capture of the tagged circular ssDNA fragments or the di-tagged linear ssDNA fragments on a bead or other surface, e.g., wherein the annealing site of the tag domain sequence permits capture by annealing to a specific sequence which is on a surface, such as a probe on a bead or on a microchip or microarray or on a sequencing bead). In some embodiments of the method, after the tagged circular ssDNA fragments or the di-tagged linear ssDNA fragments are captured by annealing to a complementary probe on a surface, the capture tag domain provides a site for priming DNA synthesis using said tagged circular ssDNA fragments or said di-tagged linear ssDNA fragments (or the complements of said tagged circular ssDNA fragments or di-tagged linear ssDNA fragments) as templates. In some other embodiments, the capture tag domain comprises a 5'-portion of the transferred strand that is joined to a chemical group or moiety that comprises or consists of an affinity binding molecule (e.g., wherein the 5'-portion of the transferred strand is joined to a first affinity binding molecule, such as biotin, streptavidin, an antigen, or an antibody that binds the antigen, that permits capture of the circular tagged ssDNA fragments or the di-tagged linear ssDNA fragments on a surface to which a second affinity binding molecule is attached that forms a specific binding pair with the first affinity binding molecule).

As used herein, an "amplification tag domain" means a tag domain that exhibits a sequence for the purpose of facilitating amplification of a nucleic acid to which said tag is appended. For example, in some embodiments, the amplification tag domain provides a priming site for a nucleic acid amplification reaction using a DNA polymerase (e.g., a PCR amplification reaction or a strand-displacement amplification reaction, or a rolling circle amplification reaction), or a ligation template for ligation of probes using a template-dependent ligase in a nucleic acid amplification reaction (e.g., a ligation chain reaction).

As used herein, a "detection tag domain" or a "detection tag" means a tag domain that exhibits a sequence or a detectable chemical or biochemical moiety for the purpose of facilitating detection of the tagged circular ssDNA fragments or the di-tagged linear ssDNA fragments (e.g., wherein the sequence or chemical moiety comprises or is joined to a detectable molecule; such as a detectable molecule selected from among: a visible, fluorescent, chemiluminescent, or other detectable dye; an enzyme that is detectable in the presence of a substrate, e.g., an alkaline phosphatase with NBT plus BCIP or a peroxidase with a suitable substrate); a detectable protein, e.g., a green fluorescent protein; and an affinity-binding molecule that is bound to a detectable moiety or that can form an affinity binding pair or a specific binding pair with another detectable affinity-binding molecule; or any of the many other detectable molecules or systems known in the art).

As used herein, an "address tag domain" or an "address tag" means a tag domain that exhibits a sequence that permits identification of a specific sample (e.g., wherein the 5'-end portion of the first-strand cDNA synthesis primer has a different address tag domain that exhibits a different sequence for each sample).

A "transposon end domain," is a tag portion or tag domain that exhibits a transferred transposon end sequence. In general, a linear ssDNA molecule that has a tag comprising a transposon end domain is generated using the Nextera™ Sample Prep Kit from EPICENTRE Biotechnologies, Madison, Wisconsin, USA, from whom information is available.

The names and descriptions of different tag domains are for convenience, such as to make it easier to understand and discuss the intended purposes and applications of the different portions or domains of the tag in different embodiments. However, these names and descriptions are not intended to limit the use or applications of the tag or of any of its tag domains in any way. Thus, any particular tag or tag domain can be used for any purpose in addition to, or in place of the intended or primary purpose or application. Also, one tag domain can comprise two or more other tag domains (e.g., a sequencing tag domain can comprise both a capture tag domain and an amplification tag domain) or one tag domain can provide the functions or purposes or applications of two or more different tag domains (e.g., a capture tag domain can also provide the function or purpose of a sequencing tag domain and/or an amplification tag domain for a particular application). Still further, the tag need not be described in terms of one or more different domains in order to be used for any particular purpose or application or function.

### GENERAL DESCRIPTION OF THE INVENTION

Circular ssDNA molecules can be made by ligation of linear single-stranded DNA (ssDNA) molecules using either a template-dependent (or homologous ligase) or a template-independent (or non-homologous) ligase.

As used herein, a "template-dependent ligase" or "homologous ligase" means a DNA ligase that catalyzes intramolecular ligation (i.e., circularization) of a linear ssDNA molecule when both ends of the ssDNA molecule to be ligated are adjacent to each other when annealed to a complementary polynucleotide. The polynucleotide to which both ends of the ssDNA molecule to be ligated anneal adjacently is referred to herein as a "ligation template" and the ligation is referred to as "template-dependent ligation" or "homologous ligation." The ligation template can be a complementary DNA sequence in genomic or other DNA in a biological sample or the ligation template can be a "bridging oligodeoxyribonucleotide" or "ligation splint oligodeoxyribonucleotide" (or "ligation splint") that is synthesized and provided for ligation of a specific or particular linear ssDNA molecule. Each bridging oligodeoxribonucleotide is designed to exhibit a nucleotide sequence which is complementary to the ends of a linear ssDNA molecule that one desires to ligate, so that the ends of the linear ssDNA molecule are adjacent when annealed to the bridging oligodeoxyribonucleotide. Examples of template-dependent DNA ligases include NAD-type DNA ligases such as *E. coli* DNA ligase, Tth DNA ligase, Tfl DNA ligase, and AMPLIGASE® DNA ligase (EPICENTRE Biotechnologies, Madison, WI, USA), which catalyze intramolecular ligation of ssDNA molecules only in the presence of a ligation template, and ATP-type DNA ligases, such as T4 DNA ligase or FASTLINK™ DNA ligase (EPICENTRE Biotechnologies), which, while they do not require a ligation template for blunt-end ligation, they catalyze template-dependent ligation much more efficiently than template-independent ligation.

Template-dependent intramolecular ligation of linear ssDNA is efficient and specific in the presence of a complementary ligation template consisting of either a target sequence in a biological sample or a bridging oligodeoxyribonucleotide and is the basis for a number of methods known in the art for detecting the presence of or quantifying target nucleic acid sequences in a biological sample. However, if the goal is to circularize a large population of different ssDNA molecules, such as all first-strand cDNA molecules synthesized by oligo(dT)-primed reverse transcription of all mRNA molecules in a sample, template-dependent ligation is extremely impractical because it is very difficult to design suitable complementary bridging oligodeoxyribonucleotides for all of the ssDNA molecules to be ligated. In particular, if the sequences of the ends of the ssDNA molecules are unknown, it is extremely difficult or impossible to design or provide complementary bridging oligodeoxyribonucleotides in order to circularize a high percentage of all of the ssDNA molecules in a large population of ssDNA molecules that vary in size and nucleotide sequence. For example, in many experiments in the laboratory of the present applicants attempting to circularize linear ssDNA molecules that exhibited unknown or random 5' and/or 3' end sequences using bridging oligodeoxyribonucleotides with different template-dependent ligases and ligation reaction conditions, no more than a few percent of the linear ssDNA molecules could be ligated, even if the bridging oligodeoxyribonucleotide exhibited random nucleotide sequences of various length, or wherein one portion of the bridging oligodeoxyribonucleotide exhibited a sequence that was complementary to a known sequence at one end of the linear ssDNA substrate that we desired to ligate and another adjacent portion of its sequence consisted of different lengths of a random sequence or different lengths of a sequence comprising a universal base such as inosine. Without being bound by theory, the applicants believe that the known sequence of the bridging oligodeoxyribonucleotides annealed correctly to the complementary sequence at one end of the linear ssDNA molecules to be ligated, but the random sequence at the other end of the bridging oligodeoxyribonucleotide often was not complementary to the other end of the linear ssDNA molecules (and therefore blocked ligation). Thus, a method that uses a template-dependent ligase is not practical for many applications (e.g., for generating circular ssDNA molecules from all first-strand cDNA molecules prepared from all mRNA molecules in a sample, (e.g., for gene expression analysis), or for generating circular ssDNA molecules from denatured genomic DNA fragments (e.g., for CNV analysis).

What is needed in the art are intramolecular ligation methods that do not require a ligation template, such as a complementary target nucleic acid or bridging oligodeoxyribonucleotide, in order generate circular ssDNA molecules from linear ssDNA molecules. What is needed are intramolecular ligation methods that employ a "template-independent" or "non-homologous" ligase, by which we mean herein, a ligase that results in intramolecular ligation of linear ssDNA to generate circular ssDNA in the absence of a ligation template, such as a target nucleic acid or bridging oligodeoxyribonucleotide to which the ends of the linear ssDNA that one desires to ligate can anneal so that its ends are adjacent.

As discussed above in the above, the methods and ligation reaction mixtures which have been tried and which are known in the art for intramolecular template-independent ligation of linear ssDNA molecules to circular ssDNA molecules yield unsatisfactory variable results, depending on the sequence and length of the linear ssDNA molecules. Thus, even with thermostable RNA ligases, such as phage TS2126 RNA ligase, which yield the best intramolecular template-independent ligation results, there is an urgent unmet need in the art for improvement of the methods for intramolecular template-independent ligation and for an improved ligation reaction mixture, and kits. The best available ligation reaction mixture and ligation conditions known in the art have used As used herein, a "standard ligation reaction mixture," which herein means a ligation reaction mixture that contains 0.5 micromolar linear ssDNA substrate molecules that have 5'-phosphoryl and 3'-hydroxyl groups, 1 micromolar thermostable RNA ligase (e.g., 5 units of CIRCLIGASE™ ssDNA ligase per microliter), 50 millimolar MOPS buffer (pH 7.5), 10 millimolar KCl, 1 millimolar DTT, 5 millimolar of MgCl₂, 2.5 millimolar MnCl₂, and 50 micromolar ATP, and "standard ligation reaction conditions" mean incubation of the linear ssDNA substrate molecules in the standard ligation reaction mixture for one hour at 60 degrees C. The above standard ligation reaction mixture and standard ligation reaction conditions are identical or similar to those which are commonly used in the art for intramolecular ligation of linear ssDNA using bacteriophage TS2126 thermostable RNA ligase. EPICENTRE Biotechnologies (Madison, WI, USA) recommends these standard ligation reaction conditions for intramolecular ligation of ssDNA using CIRCLIGASE™ ssDNA ligase (e.g., see the product literature "Lit. #222), and Prokaria, and its partner companies, Matis and Prokazyme, have recommended the standard ligation reaction conditions except that they additionally recommend adding bovine serum albumin (BSA) to a final concentration of 25 ng BSA per microliter in the standard ligation reaction mixture (e.g., see the directions for "ssDNA ligation (circularization) in Product Sheet version 4.2 for THERMOPHAGE™ ssDNA ligase (Product Number Rlig 122), dated April 26, 2004, which was available from the distributors of THERMOPHAGE™ ssDNA ligase, Prokaria, Matis, or Prokazyme as of the application date of this patent application.

After the intramolecular ligation of linear ssDNA molecules using the above standard ligation reaction conditions, the reaction products are analyzed by polyacrylamide gel electrophoresis (PAGE) under denaturing conditions. Samples can be treated with exonuclease (exo I or exo III or, preferably, a cocktail of both enzymes) prior to running on a gel, which digests unligated ssDNA molecules and helps in the identification of circular ssDNA intramolecular ligation products.

During the approximately five years that the applicants and other employees of the applicants' employer, EPICENTRE Biotechnologies, have worked with and sold CIRCLIGASE™ ssDNA ligase, EPICENTRE scientists have observed that some ssDNA oligonucleotides are more readily circularized than others that have a different sequence or length using the standard ligation reaction mixture and conditions. It has also been very difficult to use the enzyme to synthesize significant yields of circular ssDNA molecules from larger linear ssDNA molecules, such as first-strand cDNA made by reverse transcription of mRNA from a biological sample. It is preferred to have all ssDNA molecules intramolecularly ligate with the same high efficiency so that the best performance in various applications is achieved.

EPICENTRE Biotechnologies has provided guidelines for improving the ligation yield for recalcitrant or difficult-to-ligate substrates. These recommendations include varying the MnCl₂ concentration or adding more CIRCLIGASE™ enzyme compared to the standard ligation reaction mixture. However for certain substrates, the yields are still less than can be achieved with more ligatable or easy-to-ligate ssDNA substrates.

During the years of work with CIRCLIGASE™ ssDNA ligase, the applicants have also observed that different preparations of this enzyme produced variable yields of circular ssDNA products with some linear ssDNA substrates.

In 2008, EPICENTRE Biotechnologies' scientists constructed a new recombinant plasmid clone of the bacteriophage TS2126 thermostable RNA ligase gene in order to obtain better expression and yields of the protein in *Escherichia coli* cells than we had been able to obtain with a previous clone. The new recombinant clone contained the nucleotide sequence of the bacteriophage TS2126 thermostable RNA ligase gene without a hexahistidine tag as disclosed in U.S. Patent No. 7,303,901. The CIRCLIGASE™ enzyme was expressed from this new clone in good yield and had the expected molecular weight of 43,876 Daltons when it was purified and the purified enzyme preparation was analyzed by electrophoresis on a 10% polyacrylamide gel containing SDS. However, the applicants were surprised to find that the CIRCLIGASE™ enzyme prepared from this new clone consisted of mostly the adenylated form of the TS2126 thermostable RNA ligase (estimated to be approximately 70% adenylated) (Fig. 1). In contrast, similar SDS-PAGE analysis of the enzyme prepared from a previous clone showed that it consisted mostly of the non-adenylated form of the TS2126 thermostable RNA ligase (estimated to be only approximately 30% adenylated). When the enzyme preparations from both the new clone and the old clone were used under standard ligation reaction conditions (described above), lower yields of circular ssDNA ligation products were obtained using the enzyme preparation from the new clone compared to the yields obtained using the enzyme preparation from the old clone. This result was somewhat surprising and led the applicants to systematically compare the intramolecular ligation activities of the CIRCLIGASE™ enzymes prepared from both the new clone and the old clone using different linear ssDNA substrates, different ligation reaction mixtures, including ligation reaction mixtures with different levels of ATP, MgCl₂, MnCl₂, and different levels of the CIRCLIGASE™ enzymes prepared from the new and the old clone, and with different ligation reaction conditions, including different reaction times, and certain totally new components in the ligation reaction mixture, such as the zwitterionic compound trimethylglycine (betaine).

Surprisingly and unexpectedly, the applicants observed that, when the ligation reactions were carried out in the absence of ATP and with a molar excess of the adenylated form of the CIRCLIGASE™ ssDNA ligase (TS2126 thermostable RNA ligase) enzyme over linear ssDNA substrate, especially the highly adenylated enzyme from the new clone produced approximately quantitative intramolecular ligation with a variety of different linear ssDNA substrates with different nucleotide sequences and sizes (especially after the applicants found ways to further modify the ligation reactions so as to make improved ligation reaction mixtures and improved ligation reaction conditions) .

The results obtained (see EXAMPLES) enabled the applicants to develop an improved ligation reaction mixtures that permits much more efficient and consistent intramolecular ligation of linear ssDNA substrates having different nucleotide sequences and/or sizes and improved intramolecular ligation methods using said improved ligation reaction mixtures.

Thus, one embodiment of the present invention is an improved ligation reaction mixture for intramolecular ligation of linear ssDNA molecules to circular ssDNA molecules. An "improved ligation reaction mixture" herein means a ligation reaction mixture that comprises:
(a) the linear ssDNA molecules;
(b) a composition of thermostable RNA ligase molecules, wherein a high proportion of the thermostable RNA ligase molecules are adenylated and wherein the concentration of the adenylated thermostable RNA ligase molecules at least equals the molarity of the ssDNA molecules;
(c) a buffer that maintains the pH; and
(d) Mn²⁺ cations;
wherein, ATP is either not present in the reaction buffer or is present at a molar concentration that is less than the concentration of the non-adenylated form of the thermostable RNA ligase. In preferred embodiments, no ATP is added to the ligation reaction mixture.

By the statement that "a high proportion of the thermostable RNA ligase molecules are adenylated", we mean that at least approximately 60% of all of the thermostable RNA ligase molecules in the improved ligation reaction mixture are adenylated. In some embodiments of the improved ligation reaction mixture, greater than approximately 70% of all of the thermostable RNA ligase molecules are adenylated. In some embodiments of the improved ligation reaction mixture, greater than approximately 80% of all of the thermostable RNA ligase molecules are adenylated. In some preferred embodiments of the improved ligation reaction mixture, greater than approximately 90% of all of the thermostable RNA ligase molecules are adenylated. In some preferred embodiments of the improved ligation reaction mixture, greater than approximately 95% of all of the thermostable RNA ligase molecules are adenylated. In some preferred embodiments, the thermostable RNA ligase is adenylated in order to make a composition wherein a high proportion of the thermostable RNA ligase molecules are adenylated by incubating the enzyme with ATP during or after the purification process. For example, one protocol that can be used to adenylate the thermostable RNA ligase is to incubate the enzyme in a solution containing 50 mM Tris-HCl, pH 8.0, 2 mM MgCl₂, 100 mM NaCl, and 0.5 mM ATP for 15 minutes at 50 degrees C; then stop the reaction by adding EDTA to a final concentration of 5 mM; and then to remove the reaction components by dialysis or gel filtration. The percent of adenylated thermostable RNA ligase can be estimated by SDS-PAGE analysis as described in the EXAMPLES. In some preferred embodiments, the thermostable RNA ligase wherein a high proportion of the thermostable RNA ligase molecules are adenylated is bacteriophage TS2126 thermostable RNA ligase. In some embodiments of the improved ligation reaction mixture, the buffer maintains the pH at between pH 6.5 and 8.0. In some preferred embodiments of the improved ligation reaction mixture, the buffer maintains the pH at between pH 7.0 and 8.0. In some preferred embodiments of the improved ligation reaction mixture, the buffer that maintains the pH at between pH 7.0 and 8.0 is a Tris buffer. In some embodiments of the improved ligation reaction mixture, the concentration of Mn²⁺ cations is between 0.5 and 10 mM. In some embodiments of the improved ligation reaction mixture, the concentration of Mn²⁺ cations is between 1 and 10 mM. In some embodiments of the improved ligation reaction mixture, the concentration of Mn²⁺ cations is between 1 and 5 mM. In some preferred embodiments of the improved ligation reaction mixture, the concentration of Mn²⁺ cations is 2.5 mM. In some preferred embodiments of the improved ligation reaction mixture, the Mn²⁺ cations are provided as MnCl₂. In some embodiments, the concentration of the adenylated thermostable RNA ligase molecules in the improved ligation reaction mixture is at least two-fold the molarity of the ssDNA molecules. In some embodiments, the concentration of the adenylated thermostable RNA ligase molecules in the improved ligation reaction mixture is at least five-fold the molarity of the ssDNA molecules. In some embodiments, the concentration of the adenylated thermostable RNA ligase molecules in the improved ligation reaction mixture is at least ten-fold the molarity of the ssDNA molecules. In some preferred embodiments, the improved ligation reaction mixture additionally comprises a salt such as potassium chloride or potassium acetate (e.g., at a concentration of about 50 to about 100 mM). In some preferred embodiments, the improved ligation reaction mixture additionally comprises a reducing reagent such as dithiothreitol (DTT) (e.g., at a concentration of about 0.5 or 1 mM). In some embodiments, the improved ligation reaction mixture additionally comprises zwitterionic trimethyl glycine (betaine) at a concentration between 0.25 and 5.2 M. In some embodiments, the improved ligation reaction mixture additionally comprises zwitterionic trimethyl glycine (betaine) at a concentration between 0.5 and 2 M. In some embodiments, the improved ligation reaction mixture additionally comprises zwitterionic trimethyl glycine (betaine) at a concentration of about 1 M.

In some preferred embodiments, the improved ligation reaction mixture comprises:
(a) the ssDNA molecules that have 5'-phosphoryl and 3'-hydroxyl groups (e.g., 0.5 micromolar);
(b) a composition of thermostable RNA ligase molecules, wherein >70% of the thermostable RNA ligase molecules are adenylated and wherein the concentration of the adenylated thermostable RNA ligase molecules in the improved ligation reaction mixture at least equals the concentration of the ssDNA molecules (e.g., about 1 micromolar of adenylated thermostable RNA ligase for 0.5 micromolar of the linear ssDNA molecules);
(c) 33 mM TRIS acetate at a final pH of about pH 7.8; and
(d) Mn²⁺ cations at a final concentration of about 2.5 mM;
wherein, ATP is either not present in the reaction buffer or is present at a molar concentration that is less than the concentration of the non-adenylated form of the thermostable RNA ligase. In preferred embodiments, no ATP is added to the ligation reaction mixture.

In some preferred embodiments, the improved ligation reaction mixture additionally comprises 66 mM potassium acetate and 0.5 mM DTT. In some preferred embodiments, the improved ligation reaction mixture additionally comprises 1 M betaine.

Another embodiment of the invention is a method for improved intramolecular ligation of linear ssDNA molecules to synthesize circular ssDNA molecules, wherein the method comprises incubating the linear ssDNA molecules in the improved ligation reaction mixture at a reaction temperature between about 40 degrees C and about 70 degrees C for sufficient time wherein circular ssDNA molecules are synthesized. In some embodiments of the method for improved intramolecular ligation of linear ssDNA molecules to synthesize circular ssDNA molecules, the method comprises incubating the linear ssDNA molecules in the improved ligation reaction mixture at a reaction temperature between 55 degrees C and 70 degrees C for sufficient time wherein circular ssDNA molecules are synthesized. In some preferred embodiments of the method for improved intramolecular ligation of linear ssDNA molecules to synthesize circular ssDNA molecules, the method comprises incubating the linear ssDNA molecules in the improved ligation reaction mixture at a reaction temperature between 55 degrees C and 65 degrees C for sufficient time wherein circular ssDNA molecules are synthesized. In some preferred embodiments of the method for improved intramolecular ligation of linear ssDNA molecules to synthesize circular ssDNA molecules, the method comprises incubating the linear ssDNA molecules in the improved ligation reaction mixture at a reaction temperature of 60 degrees C for sufficient time wherein circular ssDNA molecules are synthesized. In some embodiments wherein the method for improved intramolecular ligation of linear ssDNA molecules is used to synthesize circular ssDNA molecules, the yield of circular ssDNA molecules synthesized is at least two-fold higher than the yield of circular ssDNA molecules synthesized from the same amount of linear ssDNA molecules using standard ligation conditions as defined herein. In some embodiments wherein the method for improved intramolecular ligation of linear ssDNA molecules is used to synthesize circular ssDNA molecules, the yield of circular ssDNA molecules synthesized is at least five-fold higher than the yield of circular ssDNA molecules synthesized from the same amount of linear ssDNA molecules using standard ligation conditions as defined herein. In some embodiments wherein the method for improved intramolecular ligation of linear ssDNA molecules is used to synthesize circular ssDNA molecules, the yield of circular ssDNA molecules synthesized is at least ten-fold higher than the yield of circular ssDNA molecules synthesized from the same amount of linear ssDNA molecules using standard ligation reaction conditions as defined herein.

The unexpected finding described above led the applicants to think about the basis for the improved ligation reaction conditions, with the hope that a better understanding of the mechanisms involved would enable us to apply and extend our findings to other situations, applications and methods. We hope our findings and better understanding will result in more efficient and consistent intramolecular ligation of linear ssDNA molecules and maximization of yields in the synthesis of circular ssDNA molecules for any application in which they are useful. Without being bound by theory or speculation, the applicants offer their thoughts and ideas below related to the basis for and the potential benefits of the inventions described herein.

In order to solve the problem of variable intramolecular ligation efficiencies of ssDNA molecules having different sequences and sizes using bacteriophage TS2126 thermostable RNA ligase, the applicants carefully considered the various catalytic steps involved in the ligation mechanism of this enzyme, which catalytic steps are common for virtually all ATP-dependent DNA and RNA ligases.

The bacteriophage TS2126 thermostable RNA ligase (e.g., CIRCLIGASE™ or THERMOPHAGE™) ligation reaction steps can be written as follows:
Reaction 1. Ligase + ATP → Adenylated Ligase + PPi
Reaction 2. Adenylated Ligase + linear 5'-pDNA → AppDNA + Ligase
Reaction 3. Ligase + AppDNA → circular DNA + AMP + Ligase

Thus, the first reaction step comprises adenylation of the epsilon amino group of the TS2126 thermostable RNA ligase active site lysine by ATP to form adenylated thermostable RNA ligase. Then, in the second reaction step, the AMP moiety is transferred from the adenylated thermostable RNA ligase to the 5'-phosphoryl group of the linear ssDNA to synthesize 5'-adenylated ssDNA. Finally, the non-adenylated thermostable RNA ligase facilitates attack of the 3'-hydroxyl group (called the "acceptor" in the ligation reaction) of the ssDNA on the 5'-adenylated end (called the "donor" in the ligation reaction), thereby joining the 3'-end to the 5'-end of the ssDNA to synthesize circular ssDNA and releasing the non-adenylated thermostable RNA ligase and AMP.

Based on the above 3-step ligation reaction mechanism for thermostable RNA ligase, one can imagine several strategies for improving the intramolecular ligation efficiency and yield of circular ssDNA molecules compared to the standard ligation reaction conditions which are used in the art.

For example, one strategy which has been employed in the art is to try to adjust the ATP concentration to find a concentration that gives more efficient or higher levels of intramolecular ligation. This strategy has sometimes been effective for optimizing intramolecular ligation of a single ssDNA substrate that is easy to ligate using the thermostable RNA ligase. By "easy to ligate", we mean a ssDNA substrate that exhibits a sequence and size that permits it to be intramolecularly ligated by the thermostable RNA ligase efficiently and to a high level to form a circular ssDNA molecule. By "a high level", we mean that at least 50% of the linear ssDNA molecules are intramolecularly ligated following one-hour incubation in the presence of the thermostable RNA ligase under standard ligation reaction conditions. However, as discussed in the "Background" section, the present applicants and other people skilled in the art, such as Nunez et al. (discussed above) have observed great differences in the intramolecular ligation efficiencies for different ssDNA molecules during a period of several years of working on this problem. In spite of our efforts to do so, we were unable to make a rule to reliably predict which ssDNA substrate would be easy to ligate and which one would by difficult or even impossible to ligate efficiently. Thus, in the situation wherein one wishes to achieve efficient or high levels of intramolecular ligation of a single difficult-to-ligate ssDNA molecule, or wherein one wishes to achieve efficient or high levels of intramolecular ligation of all ssDNA molecules in a population of ssDNA molecules that differ in sequence, size, or ligation efficiency, the strategy of trying to find a better concentration of ATP for the intramolecular ligation reaction had not been successful.

We first asked ourselves "what are the reasons why we and others had not been successful in achieving more efficient or high levels of intramolecular ligation in such situations by adjusting the ATP concentration?" In attempting to answer this, we considered the common situation of trying to achieve efficient or high levels of intramolecular ligation of all ssDNA molecules in a population of ssDNA molecules that differ in sequence, size, or ligation efficiency (such as one would encounter in trying to intramolecularly ligate all of the first-strand cDNA molecules made by reverse transcription of all mRNA molecules in a biological sample or all ssDNA fragments obtained by denaturation of fragmented dsDNA, such as denatured fragmented genomic or mitochondrial DNA). In such a population, one would find some ssDNA molecules that are easy to ligate and others that are difficult to ligate. What is happening to the easy-to-ligate ssDNA molecules, the difficult-to-ligate ssDNA molecules, and the RNA ligase at various time points after starting the ligation reaction in the presence of ATP?

The first reaction step comprising adenylation of the RNA ligase molecules begins immediately upon incubation of the ligation reaction mixture at the reaction temperature (typically at about 60 degrees C for the TS2126 RNA ligase). As this step occurs, the adenylated RNA ligase molecules are used in the second reaction step to adenylate the 5'-phosphoryl ends of the ssDNA molecules. For the easy-to-ligate ssDNA ligation substrates, meaning the ssDNA substrates that are rapidly intramolecularly ligated by the RNA ligase, the substrate may be adenylated rapidly, before all of the RNA ligase molecules are adenylated by the ATP, and then, the adenylated ssDNA molecules which remain bound to the non-adenylated ligase after the second reaction step are intramolecularly ligated by the non-adenylated RNA ligase in the third reaction step. With respect to the difficult-to-ligate ssDNA ligation substrates, meaning the substrates that are intramolecularly ligated by the RNA ligase only very slowly, either the second or the third step of the ligation reaction, or both steps, occur more slowly. Thus, as more and more of the RNA ligase molecules are adenylated by the ATP until most or all of the RNA ligase molecules are adenylated, some of the ssDNA molecules are also adenylated. However, adenylated thermostable RNA ligase is unable to intramolecularly ligate adenylated linear ssDNA molecules. Therefore, if adenylation of the all of the RNA ligase molecules occurs after some of the difficult-to-ligate ssDNA molecules are adenylated but not yet intramolecularly ligated, it is impossible to complete ligation step 3 to convert the adenylated difficult-to-ligate linear ssDNA molecules to circular ssDNA molecules. In particular, if all of the RNA ligase molecules are adenylated and the difficult-to-ligate ssDNA molecules are adenylated but not bound to non-adenylated RNA ligase after ligation reaction step 2, those adenylated ssDNA molecules will not be ligated. Therefore, performing the intramolecular ligation reaction in the presence of ATP can limit the reaction in the case where all of the thermostable RNA ligase is adenylated and some of the linear ssDNA substrate is adenylated.

One possible solution to this problem might be to lower the concentration of ATP in the ligation reaction mixture. However, if the ATP concentration is very low, ligation reaction step 1 becomes rate limiting which also reduces the rates for second and third ligation reaction steps. During the course of the reaction, the concentration of ATP decreases and therefore the overall reaction rate is also decreasing with time. Possibly one could employ an ATP regenerating system to maintain the ATP concentration at a steady state level that does not result in complete adenylation of the ligase. However, conditions may not be optimal for all of the different linear ssDNA molecules in the reaction. The added complexity by the addition of other reaction components is also undesirable because it is more difficult to control or optimize the ligation for all of the ssDNA molecules.

In summary, if ligation reaction step 1 is fast and ligation reaction steps 2 and 3 are rate limiting, a low yield of circles will be generated no matter how long the reaction is carried out. This is because all of the ligase will be in the adenylated form under standard ligation conditions where [ATP]>>[ligase]. If both ligation reaction steps 1 and 2 are fast and ligation reaction step 3 is rate limiting, a low yield of circles will be obtained for the same reason. Thus, when using the standard ligation reaction mixture and methods known in the art for intramolecular ligation of ssDNA molecules with a thermostable RNA ligase, the easy-to-ligate ssDNA ligation substrates may be adenylated and intramolecularly ligated by RNA ligase rapidly, before all of the enzyme is adenylated by the ATP, whereas the difficult-to-ligate ssDNA ligation substrates may be adenylated, but not yet ligated prior to all of the enzyme being adenylated by the ATP, making it impossible to drive intramolecular ligation of the adenylated difficult-to-ligate ssDNA substrates to completion.

Based on these considerations, the applicants hypothesize, without being bound by theory, that intramolecular ligation is improved with the improved ligation reaction mixtures and the improved ligation reaction conditions that use a molar excess of adenylated thermostable RNA ligase over the linear ssDNA molecules and no exogenous ATP because, under these conditions, the first step of the ligation reaction is bypassed, so there is no ATP to covert non-adenylated thermostable RNA ligase to adenylated form at the same time the linear ssDNA molecules are being adenylated in the second step of the ligation reaction. Thus, under steady state conditions, adenylated RNA ligase is used only to adenylate the linear ssDNA molecules in the second step of the ligation reaction. Also, under the improved ligation reaction conditions, as the adenylation of the linear ssDNA occurs, non-adenylated thermostable RNA ligase concentration is increasing in the reaction with time, which favors intramolecular ligation of the linear ssDNA molecules to synthesize circular ssDNA molecules.

Those with knowledge in the art have not paid attention to the level of adenylation of the thermostable RNA ligase provided in a ligation reaction mixture in order to improve intramolecular ligation of ssDNA molecules. The applicants could not find any disclosures by anybody with knowledge in the art who had specified a ligation reaction mixture that contains a composition of thermostable RNA ligase molecules wherein a high proportion of the thermostable RNA ligase molecules are adenylated, or who had disclosed methods for use of such an improved ligation reaction mixture for intramolecular ligation of ssDNA molecules. Thus, greater control over the kinetics of the intramolecular ligation reaction is achieved using the improved ligation reaction mixture of the present invention that lacks ATP by providing a composition of thermostable RNA ligase molecules wherein a high proportion of the thermostable RNA ligase molecules are adenylated and wherein the adenylated thermostable RNA ligase is present in the ligation reaction mixture at a concentration that equals or exceeds the molarity of the 5'-phosphorylated ssDNA molecules to be ligated.

Still further, the improved ligation reaction mixtures and improved ligation reaction conditions of the present invention permit easier optimization of other ligation reaction parameters in order to improve the rate and yield of circular DNA for a variety of DNA samples. For example, as described above, the applicants found that intramolecular ligation yield could be improved by eliminating magnesium and including only manganese as the divalent cation in the reaction. As another example, adding betaine to the reaction also has improved yields of circular DNA. Betaine is known to destabilize G-C base pairing in nucleic acids as well as improving the stability of enzymes at elevated temperatures. Both of these effects would allow for better yields by making DNA more accessible for intramolecular ligation as well as by maintaining high enzyme activity during long incubation periods.

### EXAMPLES

The present invention is further described in the following Examples. It should be understood that these Examples, while indicating preferred embodiments of the invention, are given by way of illustration only. The invention is defined in the claims.

### EXAMPLE 1

### SDS-PAGE Analysis of Purified CIRCLIGASE™ Enzyme from a New Clone of the Bacteriophage TS2126 Thermostable RNA Ligase Gene

Adenylated CIRCLIGASE™ enzyme migrates as a slightly higher band on a 10% SDS-PAGE gel from the non-adenylated CIRCLIGASE™. Figure 1 shows a silver-stained SDS-PAGE gel of the CIRCLIGASE™ enzyme preparation obtained from a new clone. The percent adenylation is estimated by comparing the relative intensities of the adenylated and non-adenylated bands; the CIRCLIGASE™ enzyme from the new clone shown here is estimated to be composed of of about 70% adenylated form and about 30% non-adenylated form.

### EXAMPLE 2

### Comparison of Intramolecular Ligation Activity of Highly Adenylated CIRCLIGASE™ Enzyme from the New Clone versus Lowly Adenylated CIRCLIGASE™ Enzyme from the Old Clone

When assayed under standard ligation reaction conditions, the lowly adenylated CIRCLIGASE™ enzyme from the old clone converted approximately >95% of the 55-nucleotide Control Oligonucleotide, a linear ssDNA supplied with CIRCLIGASE ssDNA Ligase, to a circular ssDNA product (Figure 2). In contrast, the highly adenylated CIRCLIGASE enzyme from the new clone converted only approximately 50% of the same linear ssDNA oligonucleotide to a circular ssDNA product.

### EXAMPLE 3

### Effect of ATP Concentration on Intramolecular Ligation of Linear ssDNA by a Highly Adenylated Form of CIRCLIGASE™ ssDNA Ligase Purified from a New Clone

In the presence of 50 µM ATP, as in the standard ligation reaction mixture, approximately 50% of the 55-nucleotide ssDNA Control Oligonucleotide supplied with CIRCLIGASE ssDNA Ligase was converted to a circular ssDNA product. When ATP was omitted from the standard ligation reaction mixture, approximately >95% of the 55-nucleotide ssDNA Control Oligonucleotide was converted to a circular ssDNA product (Figure 3). The circular ssDNA product was resistant to digestion by 20 units of exonuclease I (Exo I; EPICENTRE), a single-strand- specific exonuclease that requires a free 3' end. Unligated linear ssDNA substrate was degraded by Exo I, while the circular ssDNA ligation product was resistant to Exo I digestion.

### EXAMPLE 4

### Comparison of Intramolecular Ligation Activity of a Highly Adenylated Form of CIRCLIGASE™ ssDNA Ligase with a Lowly Adenylated Form of CIRCLIGASE ssDNA Ligase Using Linear ssDNA Molecules Having Different Nucleotide Sequences and Different Sizes

CIRCLIGASE™ ssDNA Ligase (EPICENTRE) that comprises only a low percentage of the adenylated form of the enzyme exhibits variable efficiency for intramolecular ligation of different linear ssDNA oligonucleotide substrates that exhibit different nucleotide sequences or sizes. However, CIRCLIGASE ssDNA Ligase that comprises a high percentage of the adenylated form of the enzyme exhibited a much higher efficiency of intramolecular ligation of different linear ssDNA oligonucleotide substrates that exhibit different nucleotide sequences or sizes when ATP was omitted from the standard ligation reaction mixture (Table in Figure 4). While eliminating ATP from the standard ligation reaction mixture increased intramolecular ligation of most of the linear ssDNA oligonucleotides tested, some linear ssDNA substrates, such as 4N454B and pYRTP.5, were still poorly ligated under these conditions. These substrates were used to further optimize the ligation reaction conditions.

### EXAMPLE 5

### Further Optimization of Intramolecular Ligation Reaction Conditions and Development of an Improved Ligation Reaction Mixture Using a Highly Adenylated Form of CIRCLIGASE™ ssDNA Ligase.

The effects of magnesium and manganese divalent cations on intramolecular ligation were evaluated using the linear ssDNA oligonucleotide pYRTP.5 substrate that was poorly ligated in EXAMPLE 4 and the highly adenylated form of CIRCLIGASE™ ssDNA Ligase (purified from the new clone). Intramolecular ligation experiments were performed using the pYRTP.5 ssDNA substrate and the highly adenylated form of CIRCLIGASE ssDNA Ligase in a new ligation reaction mixture consisting of 33 mM Tris-acetate pH 7.6, 66 mM KOAc, 0.5 mM DTT, and 0, 1, 2.5, 5, or 10 mM MnCl₂ or Mg(OAc)₂. As shown in Figure 5, the optimal ligation reaction mixture for intramolecular ligation of the linear pYRTP.5 ssDNA substrate was found to contain 2.5 mM MnCl₂ in the absence of magnesium cations. Under these ligation reaction conditions, approximately 30% of the linear pYRTP.5 ssDNA substrate was intramolecularly ligated. In addition, unlike the previous findings in the art related to the optimal ligation reaction conditions that resulted in the standard ligation reaction mixture, the presence of magnesium cations in the ligation reaction mixture in addition to the 2.5 mM MnCl₂ did not improve ligation. When intramolecular ligation reactions were performed in the same buffer that contained 2.5 mM MnCl₂ and 5 mM Mg(OAc)₂, <10% of the linear pYRTP.5 ssDNA substrate was converted to a circular ssDNA product (data not shown).

When intramolecular ligation reactions were performed in the same ligation reaction mixture containing the linear ssDNA oligonucleotide pYRTP.5 substrate, the highly adenylated form of CIRCLIGASE™ ssDNA Ligase, and 2.5 mM MnCl₂, no further improvement in the intramolecular ligation of the pYRTP.5 ssDNA substrate was observed by the addition of either 100 µg/ml of BSA or DMSO (5-20% ^{v}/ᵥ) (data not shown). The invention is not limited to improved ligation reaction mixtures that contain Tris. For example, some other embodiments of the improved ligation reaction mixture contain a MOPS pH 7.5 buffer or another buffer.

### EXAMPLE 6

### Further Development of an Improved Ligation Reaction Mixture: Effect of Betaine Intramolecular Ligation Efficiency of Difficult-to-Ligate Substrates

The effects of zwitterionic trimethylglycine (betaine) on intramolecular ligation were evaluated using the pYRTP.5 ssDNA substrate and the highly adenylated form of CIRCLIGASE ssDNA Ligase in the improved ligation reaction mixture from EXAMPLE 5, consisting of 33 mM Tris-acetate pH 7.6, 66 mM KOAc, 0.5 mM DTT, and 2.5 mM MnCl₂, plus 1 M betaine and the reaction mixture was incubated for 16 hours at 60°C. Under these improved ligation reaction conditions, nearly 100% of the linear pYRTP.5 ssDNA substrate was converted to an exonuclease-resistant, circular ssDNA product (Figure 5). Betaine did not have a detectable effect on the rate of intramolecular ligation of easy-to-ligate linear ssDNA substrates (e.g. the linear pYGT.5 ssDNA oligonucleotide), as determined by time course analysis (data not shown). Therefore, betaine appears to enhance the intramolecular ligation of difficult-to-ligate ssDNA substrates without an inhibitory effect on easy-to-ligate linear ssDNA substrates. Thus, betaine at a concentration of between 0.25 M and 2.2 M is included in some embodiments of the improved ligation reaction mixture, and in some embodiments of the improved ligation reaction conditions, a longer reaction time at about 60 degrees C is used.

### EXAMPLE 7

### Evaluation of Substrate Size Preference of CIRCLIGASE ssDNA Ligase

Many relevant applications require the circularization of a diverse population of ssDNA substrates. To evaluate the possibility for unbiased intramolecular ligation of a population of linear ssDNA substrates with different nucleotide sequences and sizes, restriction endonuclease-cleaved and denatured genomic DNA was tested as a substrate for intramolecular ligation using the highly adenylated form of CIRCLIGASE™ ssDNA Ligase. Calf thymus DNA was completely digested with Alu I, denatured by heating, quick cooled on ice. The resulting linear ssDNA molecules exhibited a reproducible and specific banding pattern and size distribution on a denaturing PAGE gel.

The denatured Alu I-digested calf thymus ssDNA fragments were incubated with the highly adenylated form of the CIRCLIGASE enzyme under the improved ligation reaction conditions from EXAMPLE 6, which resulted in conversion of approximately 10% of the 200 ng of total ssDNA substrate to an exonuclease-resistant circular ssDNA products (Figure 7). The relative size distribution and specific banding pattern observed for the linear ssDNA molecules was preserved for the circular ssDNA molecules after intramolecular ligation, which indicates, but does not prove that there is no gross substrate bias based on size or overall sequence composition for the ssDNA fragments up to a size of about 6000 nucleotides (Figure 7). Similar results were obtained with Rsa I-cleaved and then denatured genomic DNA (data not shown).

### EXAMPLE 8

### Application of the Improved Ligation Reaction Mixture and Improved Ligation Reaction Conditions to Intramolecular Ligation of linear ssDNA Molecules Using Archaebacterial Thermostable RNA Ligase

The applicants cloned, expressed and purified the *Methanobacterium thermoautotrophicum* thermostable RNA ligase 1 (MthRnl) (Torchia, C et al., Nucleic Acids Res 36: 6218-6227, 2008). The improved ligation reaction mixture and improved ligation reaction conditions described in EXAMPLE 6, but containing the MthRnl enzyme that was about 50% to 60% adenylated, improved intramolecular ligation efficiency and yields of synthesis of circular ssDNA molecules from linear ssDNA molecules. The ligation efficiencies and yields were not as high with the MthRnl enzyme as with CIRCLIGASE (TS2126 RNA ligase) with a comparable level of adenylation. Some embodiments of the improved ligation reaction mixture contain a mixture of different highly adenylated thermostable RNA ligases (e.g., including the bacteriophage TS2126 RNA ligase and MthRnl).

### EXAMPLE 9

### Use of the Method to Generate Tagged Circular ssDNA Fragments as Part of a Process for Making Next-Generation Sequencing Templates

In some embodiments, the linear ssDNA molecules comprise 5'-tagged ssDNA fragments that are generated by DNA polymerase extension of a first-strand cDNA synthesis primer using RNA (e.g., mRNA or total RNA) or DNA in a sample as a template, wherein the first-strand cDNA synthesis primer comprises a 5'-end portion and a 3'-end portion, wherein the 5'-end portion consists of a tag that exhibits a sequence that is not complementary to the template, and the 3'-end portion exhibits a sequence that is complementary to the template. In some embodiments, these 5'-tagged ssDNA fragments are intramolecularly ligated using the improved ligation reaction mixture and the improved ligation method of the present invention. In some of these embodiments the tag in the first-strand cDNA synthesis primer comprises sequencing tag domains for next-generation sequencing using a particular next-generation sequencer, such as a Roche 454 Sequencer. For example, in some embodiments, step (b) of the method of the invention is performed by first heat-denaturing the linear ssDNA molecules and then circularizing them using a template-independent ligase by performing the following reaction:

| | |
|---|---|
| x | water to a final volume of 20 microliters |
| 1 microliter | 330 mM Tris-acetate pH 7.8, 660 mM KOAc |
| 1 microliter | 50 mM MnCl₂ |
| 4 microliters | 5 M Betaine |
| 10 microliters | 20 µg/ml denatured 5'-tagged fragmented DNA |
| 4 microliters | 100 U/µl TS2126 ligase (CIRCLIGASE™ II, EPICENTRE) |
| 20 microliters | Final reaction volume |

The reaction is incubated for 2 hours at 60°C. Then, the reaction products are treated with 18 units of Exo I and 20 units of Exo III for 1 hour at 37°C to eliminate non-circularized, linear DNA. The circularized products are not digested.

### EXAMPLE 10

### PCR Analysis of Tagged Circular ssDNA from EXAMPLE 9

PCR analysis is performed using PCR primers that anneal to the tag which comprises sequencing tag domains that exhibit sequences for a specific next-gen sequencer. PCR products using these PCR primers demonstrates circularization, since only ligated, tagged circular ssDNA molecules can be amplified to generate a linear dsDNA product that corresponds to the size of the circular ssDNA. The PCR reaction is carried out as follows:

| | |
|---|---|
| 21 microliters | water |
| 1 microliter | Exonuclease-treated CircLigase II reaction (1:1000 dilution) |
| 1 microliter | 5 µM PCR primer 1 (e.g., for Roche FLX 454 adapter A) |
| 1 microliter | 5 µM PCR primer 2 (e.g., for Roche FLX 454 adapter B) |
| 1 microliter | FailSafe™ DNA polymerase |
| 25 microliters | FailSafe™ 2X PCR PreMix C |
| 50 microliters | Final reaction volume |

PCR is carried out for 29 cycles, under the following conditions:

| | |
|---|---|
| 94°C | 10 sec. |
| 50°C | 10 sec. |
| 72°C | 1 min. |

Gel analysis indicates that the size range of the produced PCR products are comparable to 5'-tagged fragmented ssDNA.

Control reactions are also carried out. When CIRCLIGASE™ II ssDNA Ligase
is omitted from the ligation reaction, PCR products are not generated from the 5'-tagged linear ssDNA fragments. When either PCR primer is omitted from the PCR reaction,
no products are produced.

The fact that the PCR products have the same size distribution as the 5'-tagged linear ssDNA fragments indicates that: 1) 5'-tagged linear ssDNA fragments can be heat-denatured to yield denatured tagged linear ssDNA fragments that are substrates for template-independent ligation; and 2) the 5'-tagged linear ssDNA fragments can be efficiently converted to tagged circular ssDNA fragments without a detectable bias (as confirmed by PCR amplification after exonuclease I and exonuclease III treatment).

### EXAMPLE 11

### PCR Analysis of Tagged Circular ssDNA from Nextera^{™}-Generated Linear Tagged ssDNA Fragments

In some other embodiments, linear tagged ssDNA molecules used in the improved ligation method of the present invention comprise 5-tagged ssDNA fragments generated using the Nextera™ sample prep kit (EPICENTRE); in these embodiments, the linear tagged ssDNA molecules comprising 5-tagged ssDNA fragments are generated using Nextera from dsDNA, such as genomic dsDNA, mitochondrial dsDNA, or even double-stranded cDNA prepared from RNA. In these embodiments, linear tagged ssDNA fragments generated using the Nextera kit have a tag that comprises a transposon end tag domain and a sequencing tag domain. After denaturing the tagged dsDNA fragments generated using Nextera to obtain the 5-tagged ssDNA fragments, these linear tagged ssDNA molecules are circularized using a similar protocol to that used in EXAMPLE 9. Then, the circular products are analyzed using a protocol similar to that used in EXAMPLE 10, except that the PCR primer 1 is complementary to a transposon end sequence (e.g., to the non-transferred transposon end sequence) and PCR primer 2 is complementary to a Roche 454 sequencer adapter (e.g., to Roche FLX 454B); following the PCR, the fact that the PCR products have the same size distribution as the 5'-tagged linear ssDNA fragments indicates that: 1) the transposon end composition has efficiently 5'-tagged and fragmented the target genomic DNA; 2) the annealed complementary 5'-tagged linear ssDNA fragments have been heat-denatured to yield denatured tagged linear ssDNA fragments that are substrates for template-independent ligation; and 3) the 5'-tagged linear ssDNA fragments have been efficiently converted to tagged circular ssDNA fragments without a detectable bias (as confirmed by PCR amplification after exonuclease I and exonuclease III treatment).

The disclosure can be further described with reference to the following clauses:
1. A ligation reaction mixture for template-independent intramolecular ligation of linear ssDNA molecules comprising:
   (a) linear ssDNA molecules that have 5'-phosphoryl and 3'-hydroxyl groups;
   (b) a composition of thermostable RNA ligase molecules, wherein a high proportion of the thermostable RNA ligase molecules are adenylated and wherein the concentration of the adenylated thermostable RNA ligase molecules in the ligation reaction mixture equals or exceeds the molarity of the ssDNA molecules;
   (c) a buffer that maintains the final pH at between about pH 6.5 and about 8.0; and
   (d) a manganese salt at a concentration that is optimal for the thermostable RNA ligase, wherein the final concentration of Mn<2+> cations in the ligation reaction mixture is between about 0.5 and about 10 mM;
   wherein, ATP is either not present in the reaction buffer or is present at a molar concentration that is less than the concentration of the non-adenylated form of the thermostable RNA ligase.
2. The ligation reaction mixture of clause 1, additionally comprising betaine (zwitterionic trimethylglycine) at a final concentration of about 0.25 to about 2 molar.
3. A method for template-independent intramolecular ligation of linear ssDNA molecules that have 5'-phosphoryl and 3'-hydroxyl groups to synthesize circular ssDNA molecules comprising: (1) preparing the ligation reaction mixture of any of clause 1; and (2) incubating the linear ssDNA molecules in the ligation reaction mixture at a reaction temperature between about 40 degrees C and about 70 degrees C for sufficient time wherein circular ssDNA molecules are synthesized.
4. The method of clauses 3, wherein the template-independent thermostable RNA ligase is selected from the group consisting of: a Thermus bacteriophage RNA ligase: bacteriophage TS2126 RNA ligase; an archaebacterium RNA ligase; *Methanobacterium thermoautotrophicum* RNA ligase 1.
5. The method of clause 4, further comprising:
   (1) preparing the ligation reaction mixture comprising:
      (a) the linear ssDNA molecules that have 5'-phosphoryl and 3'-hydroxyl groups;
      (b) a composition of phage TS2126 thermostable RNA ligase, wherein >60% of the thermostable RNA ligase molecules are adenylated and are present in an amount wherein the molarity of the adenylated RNA ligase molecules in the ligation reaction mixture equals or exceeds the molarity of the linear ssDNA molecules; and
      (c) the buffering agent;
      (d) the manganese salt that provides the Mn<2+> cations at a final concentration of between about 0.5 and 10 mM; and
   (2) Incubating the ligation reaction mixture at a temperature between about 55 degrees C and about 65 degrees C for sufficient time wherein circular ssDNA molecules are generated from the linear ssDNA molecules.
6. The method of clause 5, wherein the ligation reaction mixture additionally contains betaine (zwitterionic trimethylglycine) at a final concentration of 0.25 to 2 molar.
7. The method of any of clauses 3-6, wherein the linear ssDNA molecules comprise or consist of a population of linear ssDNA molecules wherein the nucleotide sequences of the 5' or 3' ends are unknown and/or wherein the linear ssDNA molecules vary in size.
8. The method of any of clauses 3-7 wherein the linear ssDNA molecules that have 5'-phosphoryl and 3'-hydroxyl groups used in the method for intramolecular ligation comprise or consist of a population of linear first-strand cDNA molecules generated by extension of one or more first- strand cDNA synthesis primers that anneal to complementary sequences exhibited by one or more target nucleic acid molecules in a biological sample using a DNA polymerase.
9. The method of clause 8 wherein the linear first- strand cDNA molecules generated by extension of one or more first- strand cDNA synthesis primers are further purified by removing the target nucleic acid molecules using a nuclease that specifically digests the target nucleic acid molecules but not the linear first-strand cDNA molecules, or by selectively purifying the linear first-strand cDNA molecules from the target nucleic acid molecules by incorporating an affinity tag into the linear first-strand cDNA molecules and pulling them out using an affinity binding substance with which the affinity tag forms a specific binding pair, which affinity binding substance is attached to a surface.
10. The method of clause 8 wherein each of the one or more first-strand cDNA synthesis primers comprises: a 5 '-end portion comprising or consisting of a tag that exhibits a sequence that is not substantially complementary to a sequence in the target nucleic acid molecules; and a 3 '-end portion that exhibits a sequence that is complementary to a sequence exhibited by the at least one target nucleic acid molecules from a biological sample
11. The method of clause 10 wherein the tag in the 5 '-end portion of the one or more first- strand cDNA synthesis primers comprises or consists of one or more tag domains selected from the group consisting of: an RNA polymerase promoter tag domain that exhibits a sense promoter sequence; cleavage site tag domains; sequence- specific sequencing tag domains; capture tag domains; amplification tag domains; detection tag domains; and address tag domains.
12. The method of clause 11 wherein the first-strand cDNA synthesis primers comprise or consist of a tag comprising one or more different tag domains, the one or more first-strand cDNA synthesis primers each contains a cleavage site between the tag domains of the first-strand cDNA or between its 5 '-end portion and its 3 '-end portion, and the method further comprises step (3) of: linearizing the circular first- strand cDNA molecules at the cleavage site to obtain linear first- strand cDNA molecules that each exhibit the sequence of the 3 '-end portion of the first-strand cDNA synthesis primer at its 5' end and the 5 '-end portion of the first-strand cDNA synthesis primer at its 3' end.
13. The method of clause 12 wherein the 5 '-end portion of the one or more first-strand cDNA synthesis primers comprises or consists of a tag that comprises or consists of an RNA polymerase promoter tag domain that exhibits a sense promoter sequence and a cleavage site that is 3 '-of the sense promoter sequence, and wherein the method further comprises the substeps of (i) annealing an oligodeoxyribonucleotide that exhibits an anti-sense promoter sequence to the sense promoter sequence at the [Upsilon] end of each of the linear first-strand cDNA molecules from step (3) to generate transcription substrates, and then (ii) transcribing the transcription substrates using an RNA polymerase that binds to the double- stranded RNA polymerase promoter and initiates transcription therefrom.
14. The method of clause 13, wherein, prior to substep (ii), the method further comprises the substep of generating double-stranded cDNA by extension of said oligodeoxyribonucleotide that exhibits an anti-sense promoter sequence using a DNA polymerase.
15. The method of clause 12 wherein the 5 '-end portion of the one or more first-strand cDNA synthesis primers comprises or consists of a tag that comprises or consists of two sequencer- specific sequencing tag domains, and step (3) generates linear ssDNA sequencing templates that have one sequencing tag domain on each of the 5'- and 3'-ends (di-tagged sequencing templates).
16. The method of clause 15, wherein the linear ssDNA sequencing templates that have one sequencing tag domain on each of the 5'- and 3 '-ends are used as templates for sequencing on the next-generation sequencer for which the sequencing tag domains are specific.
17. The method of any of clauses 3-11, further comprising amplifying the circular ssDNA molecules by a method selected from among: rolling circle replication, rolling circle transcription, and PCR.
18. The method of any of clauses 3-11, further comprising using the circular ssDNA molecules as templates for massively parallel sequencing.

## Claims

1. A ligation reaction mixture for template-independent intramolecular ligation of linear ssDNA molecules comprising:
(a) linear ssDNA molecules that have 5'-phosphoryl and 3'-hydroxyl groups;
(b) a composition of thermostable RNA ligase molecules, wherein a high proportion of the thermostable RNA ligase molecules are adenylated and wherein the concentration of the adenylated thermostable RNA ligase molecules in the ligation reaction mixture equals or exceeds the molarity of the ssDNA molecules;
(c) a buffer that maintains the final pH at between about pH 6.5 and about 8.0; and
(d) a manganese salt at a concentration that is optimal for the thermostable RNA ligase, wherein the final concentration of Mn²⁺ cations in the ligation reaction mixture is between about 0.5 and about 10 mM;
wherein, ATP is either not present in the reaction buffer or is present at a molar concentration that is less than the concentration of the non-adenylated form of the thermostable RNA ligase.
and additionally comprising betaine (zwitterionic trimethylglycine) at a final concentration of about 0.25 to about 2 molar.

2. The mixture of claim 1, wherein the linear ssDNA molecules comprise or consist of a population of linear ssDNA molecules wherein the nucleotide sequences of the 5' or 3' ends are unknown and/or wherein the linear ssDNA molecules vary in size.

3. A method for template-independent intramolecular ligation of linear ssDNA molecules that have 5'-phosphoryl and 3'-hydroxyl groups to synthesize circular ssDNA molecules comprising: (1) preparing the ligation reaction mixture of claim 1; and (2) incubating the linear ssDNA molecules in the ligation reaction mixture at a reaction temperature between about 40 degrees C and about 70 degrees C for sufficient time wherein circular ssDNA molecules are synthesized.

4. The mixture or the method of any preceding claim, wherein the template-independent thermostable RNA ligase is selected from the group consisting of: a *Thermus* bacteriophage RNA ligase: bacteriophage TS2126 RNA ligase; an archaebacterium RNA ligase; *Methanobacterium thermoautotrophicum* RNA ligase 1.

5. The method of claim 4, further comprising:
(1) preparing the ligation reaction mixture comprising:
(a) the linear ssDNA molecules that have 5'-phosphoryl and 3'-hydroxyl groups;
(b) a composition of phage TS2126 thermostable RNA ligase, wherein ≥60% of the thermostable RNA ligase molecules are adenylated and are present in an amount wherein the molarity of the adenylated RNA ligase molecules in the ligation reaction mixture equals or exceeds the molarity of the linear ssDNA molecules; and
(c) the buffering agent;
(d) the manganese salt that provides the Mn²⁺ cations at a final concentration of between about 0.5 and 10 mM; and
(2) Incubating the ligation reaction mixture at a temperature between about 55 degrees C and about 65 degrees C for sufficient time wherein circular ssDNA molecules are generated from the linear ssDNA molecules.

6. The mixture or method of any preceding claim, wherein the linear ssDNA molecules comprise or consist of a population of linear ssDNA molecules wherein the nucleotide sequences of the 5' or 3' ends are unknown and/or wherein the linear ssDNA molecules vary in size.

7. The mixture or method of any preceding claim wherein the linear ssDNA molecules that have 5'-phosphoryl and 3'-hydroxyl groups used in the method for intramolecular ligation comprise or consist of a population of linear first-strand cDNA molecules generated by extension of one or more first- strand cDNA synthesis primers that anneal to complementary sequences exhibited by one or more target nucleic acid molecules in a biological sample using a DNA polymerase.

8. The mixture or method of claim 7 wherein the linear first- strand cDNA molecules generated by extension of one or more first- strand cDNA synthesis primers are further purified by removing the target nucleic acid molecules using a nuclease that specifically digests the target nucleic acid molecules but not the linear first-strand cDNA molecules, or by selectively purifying the linear first-strand cDNA molecules from the target nucleic acid molecules by incorporating an affinity tag into the linear first-strand cDNA molecules and pulling them out using an affinity binding substance with which the affinity tag forms a specific binding pair, which affinity binding substance is attached to a surface.

9. The mixture or method of claim 7 wherein each of the one or more first-strand cDNA synthesis primers comprises: a 5 '-end portion comprising or consisting of a tag that exhibits a sequence that is not substantially complementary to a sequence in the target nucleic acid molecules; and a 3 '-end portion that exhibits a sequence that is complementary to a sequence exhibited by the at least one target nucleic acid molecules from a biological sample.

10. The mixture or method of claim 9 wherein the tag in the 5'-end portion of the one or more first-strand cDNA synthesis primers comprises or consists of one or more tag domains selected from the group consisting of: an RNA polymerase promoter tag domain that exhibits a sense promoter sequence; cleavage site tag domains; sequence- specific sequencing tag domains; capture tag domains; amplification tag domains; detection tag domains; and address tag domains.

11. The mixture or method of claim 10 wherein the first-strand cDNA synthesis primers comprise or consist of a tag comprising one or more different tag domains, the one or more first-strand cDNA synthesis primers each contains a cleavage site between the tag domains of the first-strand cDNA or between its 5 '-end portion and its 3 '-end portion, and the method further comprises step (3) of: linearizing the circular first- strand cDNA molecules at the cleavage site to obtain linear first- strand cDNA molecules that each exhibit the sequence of the 3 '-end portion of the first-strand cDNA synthesis primer at its 5' end and the 5 '-end portion of the first-strand cDNA synthesis primer at its 3' end.

12. The mixture or method of claim 11 wherein the 5 '-end portion of the one or more first-strand cDNA synthesis primers comprises or consists of a tag that comprises or consists of an RNA polymerase promoter tag domain that exhibits a sense promoter sequence and a cleavage site that is 3 '-of the sense promoter sequence, and wherein the method further comprises the substeps of (i) annealing an oligodeoxyribonucleotide that exhibits an anti- sense promoter sequence to the sense promoter sequence at the 3' end of each of the linear first-strand cDNA molecules from step (3) to generate transcription substrates, and then (ii) transcribing the transcription substrates using an RNA polymerase that binds to the double- stranded RNA polymerase promoter and initiates transcription therefrom.

13. The method of claim 12, wherein, prior to substep (ii), the method further comprises the substep of generating double-stranded cDNA by extension of said oligodeoxyribonucleotide that exhibits an anti-sense promoter sequence using a DNA polymerase.

14. The mixture or method of claim 11 wherein the 5 '-end portion of the one or more first-strand cDNA synthesis primers comprises or consists of a tag that comprises or consists of two sequencer-specific sequencing tag domains, and step (3) generates linear ssDNA sequencing templates that have one sequencing tag domain on each of the 5'- and 3'-ends (di-tagged sequencing templates).

15. The mixture or method of claim 14, wherein the linear ssDNA sequencing templates that have one sequencing tag domain on each of the 5'- and 3 '-ends are used as templates for sequencing on the next-generation sequencer for which the sequencing tag domains are specific.

16. The method of any of claims 3-10, further comprising amplifying the circular ssDNA molecules by a method selected from among: rolling circle replication, rolling circle transcription, and PCR.

17. The method of any of claims 3-10, further comprising using the circular ssDNA molecules as templates for massively parallel sequencing.

## Patentansprüche

1. Ligationsreaktionsgemisch für vorlagenunabhängige intramolekulare Ligation von linearen Einzelstrang-DNA-Molekülen, Folgendes umfassend:
(a) lineare Einzelstrang-DNA-Moleküle mit 5'-Phosphoryl- und 3'-Hydroxylgruppen;
(b) eine Zusammensetzung aus thermostabilen RNA-Ligasemolekülen, wobei ein großer Anteil der thermostabilen RNA-Ligasemolekülen adenyliert ist und wobei die Konzentration der adenylierten thermostabilen RNA-Ligasemoleküle im Ligationsreaktionsgemisch der Molarität der Einzelstrang-DNA-Moleküle entspricht oder sie übersteigt;
(c) einen Puffer, der den endgültigen pH-Wert zwischen etwa 6,5 und etwa 8,0 hält; und
(d) ein Mangansalz in einer Konzentration, die optimal für die thermostabile RNA-Ligase ist, wobei die Endkonzentration der Mn²⁺-Kationen im Ligationsreaktionsgemisch zwischen etwa 0,5 und etwa 10 mM liegt;
wobei ATP entweder nicht im Reaktionspuffer vorhanden ist oder in einer molaren Konzentration vorhanden ist, die niedriger ist als die Konzentration der nichtadenylierten Form der thermostabilen RNA-Ligase;
und ferner Betain umfassend (zwitterionisches Trimethylglycin) in einer zwischen etwa 0,25- und etwa 2-molaren Endkonzentration.

2. Gemisch aus Anspruch 1, wobei die linearen Einzelstrang-DNA-Moleküle eine Population aus linearen Einzelstrang-DNA-Molekülen umfassen oder daraus bestehen, wobei die Nukleotidsequenzen der 5'- oder der 3'-Enden unbekannt sind und/oder wobei die linearen Einzelstrang-DNA-Moleküle von uneinheitlicher Größe sind.

3. Verfahren zur vorlagenunabhängigen intramolekularen Ligation von linearen Einzelstrang-DNA-Molekülen mit 5'-Phosphoryl- und 3'-Hydroxylgruppen zum Synthetisieren von zirkulären Einzelstrang-DNA-Molekülen, Folgendes umfassend: (1) Herstellen des Ligationsreaktionsgemischs aus Anspruch 1; und (2) Inkubieren der linearen Einzelstrang-DNA-Moleküle im Ligationsreaktionsgemisch bei einer Temperatur zwischen etwa 40 °C und etwa 70 °C für einen ausreichend langen Zeitraum, wobei zirkuläre Einzelstrang-DNA-Moleküle synthetisiert werden.

4. Gemisch oder Verfahren nach einem der vorhergehenden Ansprüche, wobei die vorlagenunabhängige thermostabile RNA-Ligase ausgewählt ist aus der Gruppe bestehend aus: einer RNA-Ligase des Thermus-Bakteriophagen: einer RNA-Ligase des Bakteriophagen TS2126; einer *Archaebacterium*-RNA-Ligase; der RNA-Ligase 1 des *Methanobacterium thermoautotrophicum.*

5. Verfahren nach Anspruch 4, ferner umfassend:
(1) Herstellen des Ligationsreaktionsgemischs, Folgendes umfassend:
(a) die linearen Einzelstrang-DNA-Moleküle mit 5'-Phosphoryl- und 3'-Hydroxylgruppen;
(b) eine Zusammensetzung aus der thermostabilen RNA-Ligase des Phagen TS2126, wobei ≥60 % der thermostabilen RNA-Ligase-Moleküle adenyliert sind und in einer Menge vorliegen, wobei die Molarität der adenylierten RNA-Ligase-Moleküle im Ligationsreagtionsgemisch der Molarität der linearen Einzelstrang-DNA-Moleküle entspricht oder sie übersteigt;
(c) die Puffersubstanz;
(d) das Mangansalz, das die Mn²⁺-Kationen mit einer Endkonzentration zwischen etwa 0,5 und 10 mM liefert; und
(2) Inkubieren des Ligationsreaktionsgemischs bei einer Temperatur zwischen etwa 55 °C und etwa 65 °C für einen ausreichend langen Zeitraum, wobei zirkuläre Einzelstrang-DNA-Moleküle aus den linearen Einzelstrang-DNA-Molekülen synthetisiert werden.

6. Gemisch oder Verfahren nach einem der vorhergehenden Ansprüche, wobei die linearen Einzelstrang-DNA-Moleküle eine Population aus linearen Einzelstrang-DNA-Molekülen umfassen oder daraus bestehen, wobei die Nukleotidsequenzen der 5'- oder der 3'-Enden unbekannt sind und/oder wobei die linearen Einzelstrang-DNA-Moleküle von uneinheitlicher Größe sind.

7. Gemisch oder Verfahren nach einem der vorhergehenden Ansprüche, wobei die linearen Einzelstrang-DNA-Moleküle mit 5'-Phosphoryl- und 3'-Hydroxylgruppen, die im Verfahren zur intramolekularen Ligation eingesetzt werden, eine Population aus linearen Erststrang-Komplementär-DNA-Molekülen enthalten oder daraus bestehen, hergestellt durch Verlängern eines oder mehrerer Erststrang-Komplementär-DNA-Syntheseprimer, die unter Einsatz einer DNA-Polymerase an komplementäre Sequenzen binden, welche ein oder mehrere Ziel-Nukleinsäuremoleküle in einer biologischen Probe aufweisen.

8. Gemisch oder Verfahren nach Anspruch 7, wobei die linearen Erststrang-Komplementär-DNA-Moleküle, die durch Verlängern eines oder mehrerer Erststrang-Komplementär-DNA-Syntheseprimer hergestellt worden sind, weiter gereinigt werden durch Entfernen der Ziel-Nukleinsäuremoleküle unter Einsatz einer Nuklease, die gezielt die Ziel-Nukleinsäuremoleküle, nicht aber die linearen Erststrang-Komplementär-DNA-Moleküle verdaut, oder durch selektives Bereinigen der linearen Erststrang-Komplementär-DNA-Moleküle von den Ziel-Nukleinsäuremolekülen durch Einbauen eines Affinitätstags in die linearen Erststrang-Komplementär-DNA-Moleküle und durch Herausziehen derselben unter Einsatz einer affinitätsbindenden Substanz, mit der der Affinitätstag ein spezifisches Bindungspaar formt, wobei die affinitätsbindende Substanz an einer Oberfläche angebracht ist.

9. Gemisch oder Verfahren nach Anspruch 7, wobei jeder des einen oder der mehreren Erststrang-Komplementär-DNA-Syntheseprimer Folgendes umfasst: einen 5'-Endabschnitt, welcher einen Tag enthält oder aus diesem besteht, der eine Sequenz aufweist, die im Wesentlichen nicht zu einer Sequenz in den Ziel-Nukleinsäuremolekülen komplementär ist; und einen 3'-Endabschnitt, welcher eine Sequenz aufweist, die zu einer Sequenz komplementär ist, die in wenigstens einem der Ziel-Nukleinsäuremoleküle einer biologischen Probe enthalten ist.

10. Gemisch oder Verfahren nach Anspruch 9, wobei der Tag im 5'-Endabschnitt des einen oder der mehreren Erststrang-Komplementär-DNA-Syntheseprimer eine oder mehrere Tagdomänen umfasst oder daraus besteht, die aus der Gruppe ausgewählt sind, bestehend aus: einer RNA-Polymerase-Promotertagdomäne, die eine Sinnpromotersequenz aufweist; Spaltungsstellentagdomänen; sequenzspezifische Sequenztagdomänen; Capturetagdomänen; Verstärkungstagdomänen und Adresstagdomänen.

11. Gemisch oder Verfahren nach Anspruch 10, wobei die Erststrang-Komplementär-DNA-Syntheseprimer einen Tag umfassen oder daraus bestehen, der eine oder mehrere unterschiedliche Tagdomänen umfasst oder daraus besteht, wobei jeder der einen oder mehreren Erststrang-Komplementär-DNA-Syntheseprimer eine Spaltstelle zwischen den Tagdomänen der Erststrang-Komplementär-DNA oder zwischen seinem 5'-Endabschnitt und seinem 3'-Endabschnitt aufweist, und wobei das Verfahren ferner den folgenden Schritt (3) umfasst: Linearisieren der zirkulären Erststrang-Komplementär-DNA-Moleküle an der Spaltstelle, um lineare Erststrang-Komplementär-DNA-Moleküle zu erhalten, die jeweils die Sequenz vom 3'-Endabschnitt des Erststrang-Komplementär-DNA-Syntheseprimers am 5'-Ende und die Sequenz vom 5'-Endabschnitt des Erststrang-Komplementär-DNA-Syntheseprimers am 3'-Ende aufweisen.

12. Gemisch oder Verfahren nach Anspruch 11, wobei der 5'-Endabschnitt des einen oder der mehreren Erststrang-Komplementär-DNA-Syntheseprimer einen Tag umfasst oder daraus besteht, der eine RNA-Polymerase-Promotertagdomäne umfasst oder daraus besteht, die eine Sinnpromotersequenz und eine Spaltstelle an der 3'-Seite der Sinnpromotersequenz aufweist, und wobei das Verfahren ferner die folgenden Unterschritte umfasst: (i) Binden eines Oligodesoxiribonukleotids, das eine Gegensinnpromotersequenz aufweist, an die Sinnpromotersequenz an der 3'-Seite eines jeden der linearen Erststrang-Komplementär-DNA-Moleküle aus Schritt (3), um Transkriptionssubstrate herzustellen, gefolgt von (ii) Transkribieren der Transkriptionssubstrate unter Einsatz einer RNA-Polymerase, die an den doppelsträngigen RNA-Polymerasepromoter bindet und dessen Transkription initiiert.

13. Verfahren nach Anspruch 12, wobei das Verfahren vor dem Unterschritt (ii) ferner einen Unterschritt umfasst, in dem unter Einsatz einer DNA-Polymerase doppelsträngige Komplementär-DNA durch Verlängern des Oligodesoxiribonukleotids hergestellt wird, das eine Gegensinn-Promotersequenz aufweist.

14. Gemisch oder Verfahren nach Anspruch 11, wobei der 5'-Endabschnitt des einen oder der mehreren Erststrang-Komplementär-DNA-Syntheseprimer einen Tag umfasst oder daraus besteht, der zwei sequenzerspezifische Sequenztagdomänen umfasst oder daraus besteht und wobei Schritt (3) lineare Einzelstrang-DNA-Sequenzvorlagen herstellt, die jeweils eine Sequenztagdomäne am 5'-Ende und am 3'-Ende aufweisen (doppelt getaggte Sequenzvorlagen).

15. Gemisch oder Verfahren nach Anspruch 14, wobei die linearen Einzelstrang-DNA-Sequenzvorlagen mit jeweils einer Sequenztagdomäne am 5'-Ende und am 3'-Ende als Vorlagen zum Sequenzieren der Sequenzer der nächsten Generation eingesetzt werden, wofür die Sequenztagdomains spezifisch sind.

16. Verfahren nach einem der Ansprüche 3-10, ferner das Verstärken der zirkulären Einzelstrang-DNA-Moleküle unter Einsatz eines Verfahrens umfassend, das aus Rolling-circle-Replikation, Rolling-circle-Transkription und PCR ausgewählt ist.

17. Verfahren nach einem der Ansprüche 3-10, ferner den Einsatz der zirkulären Einzelstrang-DNA-Moleküle als Vorlagen für massive Parallelsequenzierung umfassend.

## Revendications

1. Mélange de réaction de ligature pour la ligature intramoléculaire indépendante de la matrice de molécules d'ADNsb linéaire comprenant :
(a) des molécules d'ADNsb linéaire qui ont des groupes 5'-phosphoryle et 3'-hydroxyle ;
(b) une composition de molécules d'ARN ligase thermostable, dans laquelle une proportion élevée des molécules d'ARN ligase thermostable est adénylée et dans laquelle la concentration des molécules d'ARN ligase thermostable adénylées dans le mélange de réaction de ligature égale ou excède la molarité des molécules d'ADNsb ;
(c) un tampon qui maintient le pH final entre environ 6,5 et environ 8,0 ; et
(d) un sel de manganèse à une concentration qui est optimale pour l'ARN ligase thermostable, dans lequel la concentration finale de cations Mn²⁺ dans le mélange de réaction de ligature se situe entre environ 0,5 et environ 10 mM ;
dans lequel, soit l'ATP n'est pas présent dans le tampon de réaction, soit il est présent à une concentration molaire qui est inférieure à la concentration de la forme non-adénylée de l'ARN ligase thermostable
et comprenant de plus de la bétaïne (triméthylglycine zwitterionique) à une concentration finale d'environ 0,25 à environ 2 molaires.

2. Mélange selon la revendication 1, dans lequel les molécules d'ADNsb linéaire comprennent ou consistent en une population de molécules d'ADNsb linéaire dans lesquelles les séquences nucléotidiques des extrémités 5' ou 3' sont inconnues et/ou dans laquelle les molécules d'ADNsb linéaire varient en taille.

3. Procédé pour la ligature intramoléculaire indépendante de la matrice de molécules d'ADNsb linéaire qui ont des groupes 5'-phosphoryle et 3'-hydroxyle pour synthétiser des molécules d'ADNsb circulaire comprenant : (1) la préparation du mélange de réaction de ligature de la revendication 1 ; et (2) l'incubation des molécules d'ADNsb linéaire dans le mélange de réaction de ligature à une température de réaction comprise entre environ 40 °C et environ 70 °C pendant un temps suffisant où des molécules d'ADNsb circulaire sont synthétisées.

4. Mélange ou procédé selon l'une quelconque des revendications précédentes, dans lequel l'ARN ligase thermostable indépendante de la matrice est choisie dans le groupe consistant en : une ARN ligase de bactériophage *Thermus :* ARN ligase de bactériophage TS2126 ; une ARN ligase d'archaebactérie ; l'ARN ligase 1 de *Methanobacterium thermoautotrophicum.*

5. Procédé selon la revendication 4, comprenant en outre :
(1) la préparation du mélange de réaction de ligature comprenant :
(a) les molécules d'ADNsb linéaire qui ont des groupes 5'-phosphoryle et 3'-hydroxyle ;
(b) une composition d'ARN ligase thermostable du phage TS2126, dans laquelle ≥60 % des molécules d'ARN ligase thermostable sont adénylées et sont présentes dans une quantité où la molarité des molécules d'ARN ligase adénylées dans le mélange de réaction de ligature égale ou excède la molarité des molécules d'ADNsb linéaire ; et
(c) l'agent tamponnant ;
(d) le sel de manganèse qui fournit les cations Mn²⁺ à une concentration finale comprise entre environ 0,5 et 10 mM ; et
(2) l'incubation du mélange de réaction de ligature à une température comprise entre environ 55 °C et environ 65 °C pendant un temps suffisant où des molécules d'ADNsb circulaire sont générées à partir des molécules d'ADNsb linéaire.

6. Mélange ou procédé selon l'une quelconque des revendications précédentes, dans lequel les molécules d'ADNsb linéaire comprennent ou consistent en une population de molécules d'ADNsb linéaire dans lesquelles les séquences nucléotidiques des extrémités 5' ou 3' sont inconnues et/ou dans lequel les molécules d'ADNsb linéaire varient en taille.

7. Mélange ou procédé selon l'une quelconque des revendications précédentes, dans lequel les molécules d'ADNsb linéaire qui ont des groupes 5'-phosphoryle et 3'-hydroxyle utilisés dans le procédé pour la ligature intramoléculaire comprennent ou consistent en une population de molécules d'ADNc premier brin linéaire générées par extension d'une ou de plusieurs amorces de synthèse d'ADNc premier brin qui s'annèlent aux séquences complémentaires présentées par une ou plusieurs molécules d'acide nucléique cible dans un échantillon biologique en utilisant une ADN polymérase.

8. Mélange ou procédé selon la revendication 7, dans lequel les molécules d'ADNc premier brin linéaire générées par extension d'une ou de plusieurs amorces de synthèse d'ADNc premier brin sont d'avantage purifiées par élimination des molécules d'acide nucléique cible en utilisant une nucléase qui digère spécifiquement les molécules d'acide nucléique cible mais pas les molécules d'ADNc premier brin linéaire, ou par purification sélective des molécules d'ADNc premier brin linéaire des molécules d'acide nucléique cible en incorporant une étiquette d'affinité au sein des molécules d'ADNc premier brin linéaire et en les extrayant en utilisant une substance de liaison par affinité avec laquelle l'étiquette d'affinité forme une paire de liaisons spécifiques, laquelle substance de fixation par affinité est attachée à une surface.

9. Mélange ou procédé selon la revendication 7, dans lequel chacune des une ou plusieurs amorces d'ADNc premier brin comprend : une partie d'extrémité 5' comprenant ou consistant en une étiquette qui présente une séquence qui n'est essentiellement pas complémentaire à une séquence dans les molécules d'acide nucléique cible ; et une partie d'extrémité 3' qui présente une séquence qui est complémentaire à une séquence présentée par la au moins une molécule d'acide nucléique cible provenant d'un échantillon biologique.

10. Mélange ou procédé selon la revendication 9, dans lequel l'étiquette dans la partie d'extrémité 5' des une ou plusieurs amorces de synthèse d'ADNc premier brin comprend ou consiste en un ou plusieurs domaines d'étiquette choisis dans le groupe consistant en : un domaine d'étiquette de promoteur d'ARN polymérase qui présente une séquence promotrice sens ; des domaines d'étiquette de site de clivage ; des domaines d'étiquette de séquençage spécifique à la séquence ; des domaines d'étiquette de capture ; des domaines d'étiquette d'amplification ; des domaines d'étiquette de détection ; et des domaines d'étiquette d'adresse.

11. Mélange ou procédé selon la revendication 10, dans lequel les amorces de synthèse d'ADNc premier brin comprennent ou consistent en une étiquette comprenant un ou plusieurs domaines d'étiquette différents, les une ou plusieurs amorces de synthèse d'ADNc premier brin contiennent chacune un site de clivage entre les domaines d'étiquette de l'ADNc premier brin ou entre sa partie d'extrémité 5' et sa partie d'extrémité 3', et le procédé comprend en outre une étape (3) de : linéarisation des molécules d'ADNc premier brin circulaire au site de clivage pour obtenir des molécules d'ADNc premier brin linéaire qui présentent chacune la séquence de la partie d'extrémité 3' de l'amorce de synthèse d'ADNc premier brin à son extrémité 5' et la partie d'extrémité 5' de l'amorce de synthèse d'ADNc premier brin à son extrémité 3'.

12. Mélange ou procédé selon la revendication 11, dans lequel la partie d'extrémité 5' de la une ou plusieurs amorces de synthèse d'ADNc premier brin comprend ou consiste en une étiquette qui comprend ou consiste en un domaine d'étiquette de promoteur d'ARN polymérase qui présente une séquence promotrice sens et un site de clivage qui est en 3' de la séquence promotrice sens, dans lequel le procédé comprend en outre les sous-étapes de (i) l'annelage d'un oligodésoxyribonucléotide qui présente une séquence promotrice anti-sens par rapport à la séquence promotrice sens à l'extrémité 3' de chacune des molécules d'ADNc premier brin linéaire provenant de l'étape (3) pour générer des substrats de transcription, puis (ii) la transcription des substrats de transcription en utilisant une ARN polymérase qui se lie au promoteur d'ARN polymérase double brin et initie la transcription à partir de celui-ci.

13. Procédé selon la revendication 12, dans lequel, avant la sous-étape (ii), le procédé comprend en outre la sous-étape de génération d'ADNc double brin par extension dudit oligodésoxyribonucléotide qui présente une séquence promotrice anti-sens en utilisant une ADN polymérase.

14. Mélange ou procédé selon la revendication 11, dans lequel la partie d'extrémité 5' des une ou plusieurs amorces de synthèse d'ADNc premier brin comprend ou consiste en une étiquette qui comprend ou consiste en deux domaines d'étiquette de séquençage spécifique au séquenceur, et une étape (3) génère des matrices de séquençage d'ADNsb linéaire qui ont un domaine d'étiquette de séquençage sur chacune des extrémités 5' et 3' (matrices de séquençage bi-étiquetées).

15. Mélange ou procédé selon la revendication 14, dans lequel les matrices de séquençage d'ADNsb linéaire qui ont un domaine d'étiquette de séquençage sur chacune des extrémités 5' et 3' sont utilisées comme matrices pour le séquençage sur le séquenceur de génération suivante pour lequel les domaines d'étiquette de séquençage sont spécifiques.

16. Procédé selon l'une quelconque des revendications 3 à 10, comprenant en outre l'amplification des molécules d'ADNsb circulaire par un procédé choisi parmi : la réplication en cercle roulant, la transcription en cercle roulant et la PCR.

17. Procédé selon l'une quelconque des revendications 3 à 10, comprenant en outre l'utilisation de molécules d'ADNsb circulaire comme matrices pour un séquençage massivement parallèle.
